# EUROPEAN PATENT APPLICATION

(11) **EP 4 840 297 A2**
(43) Date of publication of application: **18.11.2026**
(21) Application number: 26178999.4
(22) Date of filing: 15.05.2026
(51) Int. Cl.: A01H 1/08, A01H 5/08, A01H 6/34, A01H 6/82, A01H 4/00

(54) **METHODS AND COMPOSITIONS FOR PRODUCING DI-HAPLOID PLANTS FOR ENHANCED VEGETABLE BREEDING**

(30) Priority: 15.05.2025 US 202563806060 P
(71) Applicant: Lark Seeds INTL, Pasadena, CA 91105 (US)
(72) Inventor: LUO, Yao, Davis, CA 95618 (US); DANG, Trang, Davis, CA 95616 (US)
(74) Representative: Guardian IP Consulting I/S

(57) **Abstract**

The present disclosure provides novel compositions and methods for producing di-haploid plants through embryogenesis and spontaneous chromosome doubling using anthers and ovaries/ovules. Also provided are novel methods for accelerated selection of new commercial hybrid vegetable varieties.

## Description

### RELATED APPLICATION INFORMATION

This application claims priority to U.S. Application No. 63/806,060, filed on May 15, 2025, the contents of which are herein incorporated by reference.

### FIELD

The present disclosure relates to the fields of agriculture, plant biotechnology, and plant breeding. More specifically, the disclosure relates to compositions and methods for producing di-haploid plants for plant breeding. Also provided are the produced di-haploid plants, plant parts, plant cells, plant embryos, and plant seeds. Such di-haploid plants and seeds can be directly integrated into breeding cycles immediately after production to accelerate crop improvement.

### BACKGROUND

As global population continues to grow, the demand for nutritious, high-yield vegetable crops rises, placing increasing pressure on plant breeding programs to adapt and innovate. Developing improved food crops - and improved vegetable crop varieties in particular - with desirable traits such as disease resistance, enhanced postharvest quality, and environmental adaptability is critical to meet this demand. A key challenge in modem vegetable breeding is the extended time of plant breeding cycles which leads to significant delays in new crop development. There is a serious need to develop new vegetable crop varieties with high economic value in the shortest possible time to address such demands. However, traditional vegetable breeding methods often fall short due to several inherent biologically-based limitations: 1) Conventional vegetable breeding typically requires 7-10 years to develop a genetically stable - and thereby a commercially viable - variety. This prolonged process to develop genetically unique and stable vegetable varieties significantly hinders availability of improved vegetable crops, resulting in a significant challenge to effectively respond to rapidly changing markets and environmental conditions. 2) The long time and high cost of generating genetically stable lines often leads vegetable breeders to significantly limit the introduction of new genetic material into their breeding programs. This seriously impedes their ability to incorporate novel traits, such as enhanced nutritional content or stress resistance. 3) Climate change and emerging diseases progress more quickly than traditional breeding methods can adapt to, making it difficult to introduce resistance traits in a timely manner. 4) Conventional breeding often prioritizes traits like yield or disease resistance over nutritional improvement, resulting in suboptimal advancements in consumer-focused crop quality. Therefore, while consumers and industries increasingly seek vegetable crops that are resilient, sustainable, and of superior quality, traditional breeding methods are too slow and inefficient to meet these rapidly evolving needs.

Homozygous plants (i.e., a plant with two identical copies of a gene for a particular trait) are essential for plant breeding. This is due to the primary breeding goal of creating new "pure lines" or hybrid varieties; and homozygous plants allow for predictable inheritance of desired traits by ensuring that offspring of homozygous parents will inherit the same genetics for a specific trait, across generations. Homozygous plants are crucial for maintaining consistent characteristics in any plant variety. One of the primary challenges in selecting homozygous plants for vegetable crops is the prolonged time required to produce homozygous genotypes, often necessitating 7-9 generations of self-pollination.

Haploid plants are crucial in plant breeding because they allow for the rapid creation of homozygous lines through di-haploid (Di-H) or doubled haploid (DH) pathway, significantly reducing the cost and time needed (from 7-8 generations to 1-2 generations) to develop new crop varieties by eliminating the multiple generations of inbreeding required to achieve homozygosity (Chaikam V. et. al., 2019), thus accelerating the breeding process and enabling efficient selection of desired traits. Numerous studies have investigated the production of haploid plants across various species, using *in vitro* culture of gametophytes. The two primary techniques for production of haploid embryos (haploidization) by *in vitro* gametophyte culture are androgenesis and gynogenesis. In gynogenesis, mature female gametophytes (i.e., cells from the unfertilized ovaries or ovules) are cultured on a synthetic medium to induce the development of haploid embryos, which subsequently grow into complete plants. In androgenesis, cells from immature male gametophytes (i.e., the pollen or microspores) are cultured to induce the development of haploid embryos or plants. While DH approaches would significantly enhance vegetable breeding, they are not widely adopted. Current DH methods face several bottlenecks that hinder their wide application, including strong dependence on plant genotype (i.e., some genotypes are amenable to DH methods while others not), the lack of efficient method for DH plant production, particularly in the *Cucurbitaceae* and *Solanaceae* families. Additional challenges include extended timeframes and low efficiency in embryo induction, incomplete chromosome doubling, and the necessary use of problematic chemicals such as colchicine (a toxic chemical commonly used in plant breeding to induce polyploidy, or chromosome doubling), and the associated chemical induced chimeras composed of cells of different genetic makeup during the process. In plant breeding, a chimera is an individual plant composed of cells with different genetic makeup - and useless for plant breeding. Rectifying these obstacles is critical to fully realize the potential of DH technology in commercially viable vegetable crop improvement.

Despite the intensive labor and lengthy process, DH lines exhibit stable and uniform phenotypes due to their fully homozygous genetic state, in contrast to conventionally bred vegetable lines, which often remain heterozygous at multiple loci. This homozygosity of DH plants ensures consistent expression of traits across generations, making DH lines very reliable resources for breeding programs. Their complete homozygosity is also critical for generating phenotypically consistent and high-performing hybrids. This eliminates the need of additional inbreeding generations for producing foundation seed, significantly reducing the time and cost of hybrid seed production.

Due to the long process of achieving homozygosity in traditional breeding as described above, breeders tend to recycle well-performing, already genetically-fixed lines to reduce costs and accelerate variety development. This severely limits the introduction of novel genetic materials, including wild relatives or underutilized germplasms (i.e., a collection of seeds, plants, or plant parts that contain a diverse range of genetic material). DH technology, by rapidly fixing lines in a single generation, provides a way to efficiently incorporate diverse genetic sources into breeding programs.

Modern hybrid breeding relies on heterosis, also known as "hybrid vigor", the phenomenon where the offspring produced by crossing two genetically distinct plant parents exhibit superior traits, such as increased yield, growth rate, or disease resistance, compared to either of the parent plants. Heterosis is maximized when genetically diverse and complementary parents are used. However, the typical approach of using a narrow genetic base in plant breeding significantly reduces the chances of finding new allele combinations that enhance hybrid vigor. By fixing diverse, newly introduced germplasm faster, DH technology enables breeders to explore a broader spectrum of genetic variation, leading to stronger heterosis effects.

DH technology also enables faster "pre-breeding" and trait discovery. Pre-breeding is the process of taking desirable traits from wild or un-adapted plant species and gradually incorporating these traits into domesticated crop varieties through repeated crosses. Current pre-breeding efforts using "landraces" (locally adapted, traditional plant variety that has evolved over long periods of time of cultivation within a specific geographical region, naturally adapting to local climate, soils, and farming practices, often with a high level of genetic diversity), or wild relatives are often hampered by the slow process of fixing lines of evaluation. Due to exponential expansion of the population in each generation, breeders often select lines based on current market demands or personal preferences, inadvertently discarding genetic materials that may have long-term value (e.g., disease resistance, abiotic stress tolerance, or novel quality traits). This leads to a narrowed gene pool, limiting the availability of diverse genetic combinations for hybrid breeding.

DH technology also allows the discovery of epigenetic effects, which refer to changes in gene expression that occur without altering the DNA sequence itself, essentially "turning genes on or off" through modifications like DNA methylation or histone modifications, which can be influenced by environmental factors and impact an organism's phenotype without changing the underlying genetic code. Such changes can be passed on to offspring in some cases, contributing to the interplay between genetics and environment. Crop lines made through conventional breeding often carry non-fixed alleles, making it difficult to distinguish between genetic and epigenetic contributions to a trait. For a trait to be classified as epigenetically regulated, it must be heritable independent of genetic sequence changes. This requires stable, homozygous genetic backgrounds, where the effects of DNA sequence polymorphisms are minimized, allowing epigenetic factors to be identified more clearly.

Therefore, there is a need for a more reliable, repeatable and efficient genotype-independent system to produce DH and even more preferable di-haploid plants for breeding of new vegetable crops. Such a new system would also revolutionize the current breeding process in vegetable crops by increasing the probability of identifying and implementing new genetic combinations of hybrids, as well as identifying and employing new genetic sources of important and novel traits. By improving the reliability, repeatability, and efficiency of DH and diH production systems, vegetable crop improvement could be significantly accelerated, helping to meet the growing demand for nutritious and productive varieties.

The present disclosure provides solutions to these serious limitations of DH and diH plant breeding methods.

### SUMMARY

In one embodiment, the present disclosure relates to a method for producing a di-haploid *Solanaceae* plant from microspores. The method comprises:
a) selecting one or more *Solanaceae* plant flower buds having a length of about 2 to about 6 mm;
b) excising one or more anthers containing microspores from the flower buds;
c) subjecting the anthers containing microspores to one or more stress conditions selected from the group consisting of heat treatment, osmotic treatment, or a combination of heat and osmotic treatments;
d) culturing or growing anthers containing microspores on an induction medium to allow: (i) the microspores to develop into plants; and (ii) for spontaneous chromosome doubling, wherein the chromosome doubling results in di-haploid plants;
e) selecting the di-haploid *Solanaceae* plants; and
f) growing the selected di-haploid *Solanaceae* plants to maturity and collecting seed.

In some aspects of the above method, the induction medium comprises at least: a) one or more ammonium salts; b) calcium chloride; c) boric acid; d) cupric sulfate; e) magnesium sulfate; f) manganese sulfate; g) molybdic acid (sodium salt); h) potassium nitrate; i) potassium phosphate or sodium phosphate; j) zinc sulfate; k) myo-inositol; l) nicotinic acid; m) pyridoxine HCl; n) thiamine HCl; and o) one or more plant growth regulators selected from the group consisting of: (i) 0.1-1 mg/L IAA and 0.05-2 mg/L zeatin; (ii) 0.1 mg/L 2,4-D, 0.88 mg/L IAA, and 0.55 mg/L zeatin; (iii) 0.02-5 µM 2,4-D; (iv) 5 mg/L kinetin and 5 mg/L 2,4-D; and (v) 0.01-0.5 mg/L thidiazuron (TDZ) and 1-10 mg/L zeatin..

In further aspects of the above method, the ammonium salt is ammonium nitrate or ammonium sulfate.

In still yet further aspects of the above method, the microspores are at a late uninucleate to early binucleate development stage.

In still yet further aspects of the above method, the heat treatment is carried out at about 26-40°C for 1-40 days in the dark.

In still yet further aspects, the heat treatment is carried out at about 27-37°C for 3-10 days in the dark.

In still yet further aspects of the above method, the osmotic treatment comprises supplying about 100 to about 120 g/L of sucrose to the induction medium.

In still yet other aspects of the above method, the selecting of di-haploid *Solanaceae* plants comprises using flow cytometry. In other aspects, the selecting of di-haploid *Solanaceae* plants uses genotyping.

In still yet other aspects of the above method, the *Solanaceae* plant is a tomato (*Lycopersicon)* plant, a pepper (*Capsicum)* plant, or an eggplant (*Solanum*) plant.

In another embodiment, the present disclosure relates to a di-haploid *Solanaceae* plant, plant part, plant seed, or plant embryo produced by the above method.

In yet further aspects of the above method, the medium is a liquid medium. In still other aspects, the medium is a semi-solid medium. In still yet further aspects, the medium comprises a bottom semi-solid medium layer and an upper liquid medium layer.

In yet another embodiment, the present disclosure relates to a method for producing a di-haploid *Cucurbitaceae* plant from ovaries or ovules. The method comprises:
a) selecting and collecting unfertilized ovaries from a *Cucurbitaceae* plant about six hours before anthesis;
b) slicing the ovaries into about 1-5 mm cross sections;
c) subjecting the slices to one or more stress conditions selected from the group consisting of heat treatment, osmotic treatment, or a combination of heat and osmotic treatments;
d) culturing or growing ovary slices on an induction medium to allow: (i) development into plants; and (ii) for spontaneous chromosome doubling, wherein the chromosome doubling results in di-haploid plants;
e) selecting di-haploid *Cucurbitaceae* plants; and
f) growing the selected di-haploid *Cucurbitaceae* plants to maturity and collecting seed.

In some aspects of the above method, the induction medium comprises at least a) one or more ammonium salt; b) calcium chloride; c) boric acid; d) cupric sulfate; e) magnesium sulfate; f) manganese sulfate, g) molybdic acid (sodium salt); h) potassium nitrate; i) potassium phosphate or sodium phosphate; j) zinc sulfate; k) myo-inositol; l) nicotinic acid; m) pyridoxine HCl; n) thiamine HCl; o) 0.01-0.5 mg/L thidiazuron (TDZ).

In still yet other aspects of the above method, the ammonium salt is ammonium nitrate or ammonium sulfate.

In still yet further aspects of the above method, the heat treatment is carried out at 26-40°C for 1-60 days in the dark. In still yet other aspects, the heat treatment is carried out at 27-37°C for 1-42 days in the dark.

In still yet other aspects of the above method, the selecting of regenerated di-haploid *Cucurbitaceae* plants is using flow cytometry. In other aspects, the selecting of regenerated di-haploid *Cucurbitaceae* plants is using genotyping. In still other aspects, the selecting of regenerated di-haploid *Cucurbitaceae* plants is using low coverage sequencing.

In still yet further aspects of the above method, the *Cucurbitaceae* plant is a zucchini *(Cucurbita)* plant, a cucumber *(Cucumis)* plant, a pumpkin *(Cucurbita)* plant, or a melon *(Cucumis)* plant.

In yet another embodiment, the present disclosure relates to a di-haploid *Cucurbitaceae* plant, plant part, plant seed, or plant embryo produced using ovaries or ovules as described in the above method.

In yet another embodiment, the present disclosure relates to a method for accelerated selection of new commercial hybrid vegetable varieties. The method comprises:
a) selecting elite F1 hybrid plants with broad genetic backgrounds and/or complementary agronomic traits;
b) crossing the selected F1 hybrid plants to produce F2 populations;
c) selecting top-performing F2 plants and performing whole genome sequencing to generate marker panel;
d) producing di-haploid plants from the selected F2 plants using the methods of the invention;
e) subjecting the produced di-haploid plants to genotyping using heterozygous markers generated from the marker panel;
f) selecting di-haploid lines with beneficial allele combinations using AI-assisted genomic selection;
g) crossing the selected di-haploid lines to produce hybrid candidates; and
h) validating heterosis performance in the hybrid candidates through field trials.

In some aspects of the above method, the selecting elite F1 hybrid plants in step a) involves the use of whole genome sequencing.

In yet further aspects of the above method, the agronomic trait is selected from the group consisting of yield, fruit quality, disease resistance, and stress tolerance, or any combination thereof.

### BRIEF DESCRIPTION OF THE DRAWINGS

Figure 1 shows the steps for production of doubled haploid pepper plants from microspore-derived embryos.
Figure 2 shows flow cytometric histograms of DAPI-stained leaf nuclei of diploid pepper plant (FIG. 2A) and haploid pepper plant (FIG.2B). The X-axis represents relative fluorescence, and the Y-axis represents count of events (number of cells). In other word, the height of the graph at a given point on the x-axis indicates how many cells exhibited that fluorescence intensity.
Figure 3 provides a flowchart showing a novel approach combining a four-way crossing with the di-haploid method of the present disclosure in vegetable breeding for accelerated selection and production of new commercial hybrids with higher heterosis compared to the two original parental commercial hybrids. "WGS" stands for "Whole Genome Sequencing"; "DH" stands for "double haploid".
Figure 4 shows the morphological spectrum of fruit shapes from multiple distinct eggplant market types and germplasms.
Figure 5 shows a process for production of doubled haploid cucumber plants from ovaries.
Figure 6 provides an illustration of the Fluidigm method. FIG. 6A: a schematic diagram showing the Fluidigm method. SNP [C/G] represents the site of a SNP in the genome for targeted detection; STA refers to Specific Target Amplification Primer; LSP refers to the Locus Specific Primer; ASP1 and ASP2 refer to the Allele Specific Primer 1, which contains the C SNP, and carries the FAM fluorophore; and the Allele Specific Primer 2, which contains the G SNP, and carries the HEX fluorophore. FIG. 6B: an illustrative histogram of the amplification products: XX by ASP1, and YY by ASP2.

### DETAILED DESCRIPTION

Traditional diploid plants obtain two sets (2n) of chromosomes through fertilization between female and male gametes. Haploids (1n) contain only one (e.g., a single) set of chromosomes that are found after meiosis in male or female gametes. Doubled haploids (DH) become diploid (2n) through chromosome doubling of the haploid chromosomes by chemical means (e.g., treatment with colchicine) or spontaneous doubling, and therefore, carry two identical sets of chromosomes derived from haploids. These plants are homozygous at every single locus (the specific location on a chromosome where a particular gene is found). The utilization of doubled haploids (DH) in a plant breeding program allows new inbred populations to be created from desired parents in 1-2 generations. Application of the DH technology can enable breeding to obtain pure 2n homozygous plants in a single generation, compared to six or more generations of selfing (i.e., when a plant is used to fertilize itself) or backcrossing in typical breeding schemes. DH technology provides an important tool to reduce breeding cycle time with improved heritability to accelerate genetic gain while reducing the costs associated with maintaining multiple generations of inbreeding. In addition, DH is also a useful tool to obtain homozygous plants faster in other processes, such as gene stacking, genome editing, cytoplasm and nuclear genome exchange, trait integration, etc. (Ren et al. 2017).

While DH technology offers such advantages in plant breeding, current DH methods face several bottlenecks that hinder their wide application. The first major bottleneck for methods currently known in the art is high genotype dependency - meaning a protocol effective for one genotype may fail entirely for another. Consequently, protocols optimized for one genotype often yield low efficiency or prove ineffective for others, even within the same species. Since the DH process involves multiple intricate steps - such as preparing donor plants, inducing haploids, chromosome doubling, developing embryos *in vitro,* and germinating plants, it becomes cost-, time-, and resource-prohibitive or intensive to reconfigure and optimize these DH protocols for individual genotypes. Accordingly, the present disclosure provides a novel approach that effectively overcomes the inefficiencies and limitations associated with conventional DH procedures.

In *Solanaceae* crops such as tomato (*Lycopersicon)*, pepper (*Capsicum)*, and eggplant (*Solanum*), genotype dependency manifests as variability in response to *in vitro* culture conditions, such as anther or microspore culture. For instance, while certain pepper genotypes may respond well to androgenesis (*in vitro* development of haploid plants from microspores or immature pollen grains), others, such as the hot pepper varieties "Jalapeño or "Anaheim", often fail to produce viable embryos or plants. This variability is largely attributed to differences in cellular mechanisms, including the totipotency (i.e., the ability of a single cell to develop into a complete plant) of microspores and their regeneration potential, which may require entirely distinct conditions to be effectively induced.

The second bottleneck of the currently used DH method is that the generated DH plants often result in undesirable haploid plants. The most sought-after plants are spontaneous diploids (di-haploids diH), as these plants are fertile and can be used directly in breeding programs by plant breeders, which represents an enormous advantage in terms of time, space, and cost. Haploid plants produced through these various techniques - without spontaneously chromosome doubling-must undergo an additional step to become diploid (2n), thus forming DH. This is typically achieved using chemicals such as colchicine, an alkaloid that induces chromosome doubling. However, these chemicals are often toxic, can result in mixed-ploidy plant due to the inability to precisely control the chromosome-doubling process, and frequently cause severe damage to haploid plants. As a result, current best practices focus on the generation of spontaneous diploids. It is important to note that spontaneous di-haploids (diH) can occur through the spontaneous doubling of chromosome numbers in haploid plants, although this phenomenon is rare and unpredictable under natural conditions, while DH are artificially induced to double their chromosomes. Spontaneous diH are more reliable for breeding purposes as they avoid the risks of toxicity and variability associated with chemical doubling.

The third significant bottleneck of current DH methods for DH plant production is that it is known to be a notoriously slow process, often requiring several months-to-years to produce enough DH plants for effective breeding purposes. For most breeding programs, this typically requires generating over 100 DH/diH plants per variety to achieve the desired genetic stability and diversity. The delay is primarily driven by the need for meticulously optimized protocols to address variability in genotype responsiveness. For genotypes that are recalcitrant to existing methods, these challenges can prolong timelines even further, making DH production an inefficient and resource-intensive process for vegetable crops. (Keles et al., 2015; Graozeva et al., 2021; Esin et al., 2021)

Compared to vegetable crops that complete their life cycle within a year (i.e., the annual vegetable crops), there is an even greater need to develop a genotype-independent, highly efficient double haploid system for biennial crops such as carrots and onions. These crops require two years to complete their reproductive cycle and face significant challenges in maintaining and advancing inbred lines due to severe inbreeding depression. This phenomenon leads to reduced vigor, lower fertility, and poor seed set, ultimately hindering the development of stable inbred lines. Given these challenges, DH technology offers a promising alternative by enabling the production of completely homozygous lines in a single generation, thereby bypassing the detrimental effects of prolonged selfing and inbreeding depression while enhancing breeding efficiency.

Meanwhile, the DH process can serve as an effective mechanism for eliminating detrimental genes in crops like onions and carrots, as lethal alleles become homozygous and are naturally purged from the population. Due to their highly heterozygous nature, these biennial crops often carry recessive deleterious mutations that remain hidden in heterozygous individuals. However, when subject to DH production, all loci become homozygous, which unmasks and eliminates lethal or highly deleterious alleles that would otherwise persist through conventional breeding. This natural selection during the DH process results in the development of genetically stable, vigorous lines free from such detrimental mutations. Consequently, the surviving DH lines exhibit enhanced breeding efficiency and improved genetic integrity, leading to the production of more resilient and high-performing cultivars.

The methods of the present disclosure improve the vegetable DH process, resulting in a dramatic increase in the production of healthy doubled haploid embryos/plants in a genotype-independent fashion. Additionally, the methods of the present disclosure can eliminate the need to use toxic chromosome doubling agents such as colchicine. Therefore, the methods of the present disclosure significantly improve the efficiency of a DH breeding program by scaling up the volume in the production pipeline without additional investment.

Haploid plants can be generated using in *vivo* or *in vitro* methods (Segui-Simarro et al., 2021). The *in vivo* methods include: 1) pollen irradiation, in which pollen is irradiated to damage their genetic material enough to stimulate the development of an embryo from the female egg cell without fertilization to create haploid plants; 2) wide hybridization or inter-specific hybridization for elimination of the paternal chromosomes to generate haploid plants; or 3) use of naturally occurring or transgenic or gene edited genetic mutant haploid inducer lines, in which a haploid inducer line is used to pollinate a desired plant or source germplasm, resulting in offsprings with only half the normal chromosome set (1n), essentially containing the genetic information from the female parent only.

For the *in vitro* methods, on the other hand, haploid embryos/plants can be produced by *in vitro* culture of gametophyte. This process is characterized by the gametophyte having a single set of chromosomes (haploid) and being multicellular gametes (sperm and eggs) to produce plants via mitosis, through gynogenesis or androgenesis. In gynogenesis, mature female gametophytes (i.e., cells from the unfertilized ovaries or ovules) are cultured on a synthetic culture medium to induce the development of haploid embryos, which subsequently grow into complete plants. In androgenesis, cells from immature male gametophytes (i.e., microspores or pollen grains) are cultured for the same purpose.

### Culture Media

The term "tissue culture" refers to techniques for growing plant cells, tissues, or organs on synthetic nutrient media of known compositions in sterile environment under controlled conditions to produce plants with improved characteristics. The term "synthetic nutrient media" or "nutrient culture media" or "synthetic culture media" or "plant tissue culture media", as used interchangeably herein, refers to specially formulated, chemically defined liquid or semisolid compositions to provide essential nutrients and support for plant cells, tissues, or organs to grow and development in a laboratory setting, Key components of plant tissue culture media typically include a basal salt mixture, which provides essential macro- and micronutrients (e.g., nitrogen, phosphorus, potassium, iron, zinc), carbon source, vitamins, plant growth regulators, with or without one or more solidifying agents such as agar or gelrite, which provides a physical support system, and other optional supplements such as amino acids, activated charcoal, or other organic substances. The formulation of the tissue culture media for the specific tissue and the specific plant plays a vital role in the development of plant tissue culture techniques since each plant has its own morphology and nutrient requirements, and the nutrient requirement for the tissues in each part of the plant varies.

Exemplary types of tissue cultures are protoplasts, calli, and plant cells, such as microspore cells, that are intact in plants or parts of plants, such as flowers, anthers, pollen, ovules, ovaries, embryos, umbels, leaves, stems, roots, root tips, and the like. In one embodiment, the tissue culture comprises flower buds, anthers, pollen, microspores, ovaries, ovules, embryos, or meristematic cells derived from immature tissues of these plant parts. Methods for preparing and maintaining plant tissue cultures, and for regenerating plants are known in the art.

Production of double haploid plants from microspore or ovary/ovule of the present disclosure involves first incubating the microspore or ovary/ovule on an induction medium at an elevated temperature for 1-60 days (heat pretreatment), followed by culturing at about 25°C to induce embryo formation coupled with chromosome doubling. As used herein, an "induction medium" refers to a medium containing basal salts (also known as macro- and micro-nutrients), vitamins, carbohydrates, and plant growth regulators such as cytokinins and auxins, that is used in anther/microspore or ovary culture to induce embryogenesis and development, and chromosome doubling. An induction medium may be a liquid medium overlaid on (e.g., on top of) a semi-solid medium. An induction medium may also be a semi-solid medium containing one or more solidifying agents such as gelrite or plant agar.

Various basal media may be used in combination with plant growth regulators in the induction medium to achieve embryogenesis and spontaneous chromosome doubling. The induction medium contains at least a) one or more ammonium salts such as ammonium nitrate, or ammonium sulfate; b) calcium chloride; c) boric acid; d) cupric sulfate; e) magnesium sulfate; f) manganese sulfate, g) molybdic acid (sodium salt); h) potassium nitrate; i) potassium phosphate or sodium phosphate; j) zinc sulfate; k) myo-inositol; l) nicotinic acid; m) pyridoxine HCl; and n) thiamine HCl. Exemplary basal medium for induction medium includes Nitsch & Nitsch Basal Medium with Vitamins (N&N), Murashige & Skoog Basal Medium with Vitamins (MS), Onion Basal Medium (Onion), Gamborg B-5 Basal Medium (B5), and Eggplant Basal Medium (Eggplant). The compositions of Nitsch & Nitsch Basal Medium with Vitamins, Murashige & Skoog Basal Medium with Vitamins, Onion Basal Medium, Gamborg B-5 Basal Medium, and Eggplant Basal Medium are shown in Table 1-1. Variations of or modifications to these basal salts/media, or use of other basal salts are within the scope of the present disclosure.

The induction medium must also contain one or more auxin-type plant growth regulators (such as IAA, NAA, and/or 2,4-D), and/or one or more cytokinin-type plant growth regulators (such as Zeatin, kinetin, TDZ, and/or 2iP).

**Table 1-1. Basal medium compositions for use as an induction media**

| **Medium Component** | **Concentration (mg/L)** | | | | |
|---|---|---|---|---|---|
| | **NN¹** | **MS²** | **Onion³** | **B5⁴** | **Eggplant⁵** |
| Ammonium nitrate | 720 | 1650 | 3202 | - | 1238 |
| Ammonium sulfate | - | - | 1340 | 134 | 34 |
| Boric acid | 10 | 6.2 | 300 | 3 | 3.15 |
| Calcium chloride, anhydrous | 166 | 332.2 | - | 113.24 | - |
| Calcium chloride·2H2O | - | - | 1500 | - | 313 |
| Calcium nitrate·4H2O | - | - | - | - | 50 |
| Cupric sulfate·5H₂O | 0.025 | 0.025 | 3.9 | 0.025 | 0.016 |
| Cobalt chloride·6H₂O | - | 0.025 | 2.5 | 0.025 | 0.016 |
| Na₂EDTA·2H₂O | 37.26 | 37.26 | 37.3 | 37.26 | - |
| Ferrous sulfate ·7H₂O | 27.8 | 27.8 | 27.85 | 27.7 | - |
| Magnesium sulfate, anhydrous | 90.372 | 180.7 | - | 122.09 | - |
| Magnesium sulfate 7H2O | - | - | 2470 | - | 412 |
| Manganese sulfate·4H₂O | 18.9 | 16.9 | - | 10 | 22.13 |
| Manganese sulfate·H₂O | - | - | 925 | - | - |
| Molybdic acid (sodium salt)·2H₂O | 0.25 | 0.25 | 25 | 0.25 | 0.188 |
| Potassium nitrate | 950 | 1900 | 25300 | 2500 | 2150 |
| Potassium iodide | - | 0.83 | 75 | 0.75 | 0.695 |
| Potassium chloride | - | - | - | - | 7 |
| Potassium phosphate, monobasic | 68 | 170 | - | - | 142 |
| Sodium phosphate, monobasic | - | - | - | 150 | - |
| Sodium phosphate·H2O | - | - | 1690 | - | 38 |
| Zinc sulfate ·7H₂O | 10 | 8.6 | 200 | 2 | 3.625 |
| D-Biotin | 0.05 | - | - | - | 0.005 |
| Folic acid | 0.5 | - | - | - | - |
| Glycine (free base) | 2 | 2 | - | - | - |
| Myo-inositol | 100 | 100 | 500 | 100 | 100 |
| Nicotinic acid (free acid) | 5 | 0.5 | 1 | 1 | 0.7 |
| Pyridoxine·HCl | 0.5 | 0.5 | 1 | 1 | 5.5 |
| Thiamine·HCl | 0.5 | 0.1 | 1 | 10 | 0.6 |
| Calcium panthothenate | - | - | - | - | 0.5 |
| L-proline | - | - | 200 | - | - |

| | | | | | |
|---|---|---|---|---|---|
| ¹ PhytoTech Labs, catalog number N616 ² PhytoTech Labs, catalog number M519 ³ Dunstan and Short, 1977 ⁴ PhytoTech Labs, catalog number G398 ⁵ Calabuig-Serna et al., 2021 | | | | | |

An important component of the induction medium is the plant growth regulators. The anthers or the later released microspores, or the cross-sectioned ovary segments, are in contact with a specific combination of plant growth regulators to induce embryogenesis and spontaneous chromosome doubling. The plant growth regulators include, but are not limited to, auxins, cytokinins, gibberellins, abscisic acid, and combinations thereof. The inventors of the present disclosure surprisingly found that certain combinations of the plant growth regulators increase the rate of development of normal embryos from the microspores or the ovaries, in particular, embryos competent for plant regeneration. In some embodiments, the induction medium contains one auxin and one cytokinin (such as kinetin and 2,4-D). In other embodiments, the induction medium contains two auxins and one cytokinins (such as 2,4-D and IAA, and Zeatin). In still other embodiments, the induction medium contains two cytokinins (such as TDZ and kinetin). In still other embodiments, the induction medium contains one auxin (such as 2,4-D).

Illustrative examples of auxins useful in the practice of the present disclosure include, but are not limited to, indoleacetic acid (IAA), 2,4-dichlorophenoxyacetic acid (2,4-D), indolebutyric acid (IBA), naphthaleneacetic acid (NAA), and phenylacetic acid (PAA). In one aspect, the auxin is NAA. In another aspect, the auxin is 2,4-D. Illustrative examples of cytokinins useful in the practice of the present disclosure include, but are not limited to, trans-zeatin, trans-zeatin riboside, kinetin, 6-benzyladenine (6-BA or BA), 6-(y, γ-dimethylallylamino)purine (2iP), and thidiazuron (TDZ, which also exhibit auxin like effect). In one aspect, the cytokinin is zeatin. In another aspect, the cytokinin is kinetin. In still another aspect, the cytokinin is TDZ and zeatin.

Whenever is included in plant tissue culture media, a plant growth regulator is used in an effective amount. The concentration range of the plant growth regulator used varies depending on multiple factors such as the specific plant growth regulator, the plant species, and the target tissue used for tissue culture. The concentration range may be from about 0.01 µg/L to about 10 g/L; from about 0.1 µg/L to about 10 g/L; from 0.1 µg/L to about 5 g/L; from about 0.5 µg/L to about 2.5 g/L; from about 1 µg/L to about 1 g/L; from about 5 µg/L to about 500 mg/L; from about 20 µg/L to about 250 mg/L.

Non-limiting examples of concentrations of auxins useful in the present disclosure include, but are not limited to, from about 0.1 µg/L to about 1 g/L, a range selected from one of the following: from about 1 µg/L to about 500 mg/L, from about 5 µg/L to about 250 mg/L, from about 10 µg/L to about 10 mg/L, from about 20 µg/L to about 5 mg/L IAA and/or 2,4-D. Non-limiting examples of concentrations of cytokinins useful in the present disclosure include, not are not limited to, from about 0.0005 mg/L to about 20 mg/L, from about 0.01 mg/L to 10 mg/L trans-zeatin.

Other medium components important for anther or ovary culture for embryogenesis include carbohydrates (such as one or more of maltose, sucrose, glucose, fructose, mannitol or sorbitol), vitamins, amino acids or sources of amino acids, and/or absorbent materials, such as activated charcoal, which is capable of absorbing harmful substances, phenolic compounds released by explants which can cause browning, and ethylene, a gas that can inhibit growth.

In some embodiments, the induction media contains a high level of one or more carbohydrates, e.g., about 100 to about 120 g/L of sucrose, which, in addition to providing carbon source for the plant material in tissue culture, functions as an osmoticum (e.g., osmotic treatment). As used herein, the term "osmoticum" or "osmotic treatment" refers to a medium or treatment (e.g., adding to a medium) that induces osmotic stress or loss of cellular water content. Osmotic stress can disrupt plasmodesmatal connections between pre-embryonic cells, allowing for greater differentiation and potentially higher haploid embryo development. In some aspects, the osmotic treatment involves adding from about 100 to about 120 g/L sucrose in an induction medium or providing an induction medium that contains from about 100 to about 120 g/L sucrose. In some aspects, the osmotic treatment involves adding from about 20 to about 50 g/L glucose in an induction medium or providing an induction medium that contains from about 20 to about 50 g/L glucose. In some aspects, the osmotic treatment involves adding from about 15 to about 60 g/L sorbitol in an induction medium or providing an induction medium that contains from about 15 to about 60 g/L sorbitol. In some aspects, the osmotic treatment involves adding from about 15 to about 60 g/L mannitol in an induction medium or providing an induction medium that contains from about 15 to about 60 g/L mannitol. In some aspects, the osmotic treatment involves adding two osmotica in an induction medium or providing an induction medium that contains two osmotica.

Following induction of embryos on an induction medium, the embryos are transferred to an embryo germination medium for growth and development. As used herein, "germination media" refer to media that provide a controlled environment where the induced embryos can complete their development and transition into plantlets. A germination medium contains macro- and micronutrients, vitamins, carbohydrates such as sucrose, and other additives such as amino acids, and a gelling agent such as agar or gelrite, A germination medium may also contain one or more plant growth regulators. The various germination media of the present disclosure are summarized in Table 1-2.

Embryos with fully developed cotyledons or newly formed plantlets are next transferred to a regeneration medium or rooting medium. As used herein, "regeneration media" or "rooting media" refer to media that provide a controlled environment for the plantlets to continue growth and form roots. A regeneration medium or rooting medium contains full strength or half strength macro- and micronutrients, vitamins, carbohydrates such as sucrose, and other additives such as amino acids, and a gelling agent such as agar or gelrite, A germination medium may also contain one or more plant growth regulators. The various regeneration media of the present disclosure are summarized in Table 1-3.

**Table 1-2. Compositions of germination media**

| **Pepper** | **Tomato** | **Eggplant** | **Cucumber (Gynogenesis)** | **Cucumber/Squash (Androgenesis)** | **Carrot** | **Onion** |
|---|---|---|---|---|---|---|
| MS basal salts | Gamborg B-5 basal salts | See Table 6 | Same as the induction medium | MS basal medium with vitamins | Gamborg B-5 basal salts | Dunstan and Short, 1977 |
| 3% sucrose | 3% sucrose | | | 800 mg/L L-glutamine | 500 mg/L L-glutamine | |
| 0.8% plant agar | 0.01-0.3 mg/L Zeatin | | | 1 mg/L 2,4-D | 100 mg/L L-serine | |
| | 0.8% agar | | | 0.5 mg/L BAP | 2% sucrose | |
| | | | | 100 g/L sucrose | 0.5% agar | |
| | | | | 0.7% agar | | |

**Table 1-3. Compositions of rooting media**

| **Pepper** | **Tomato** | **Eggplant** | **Cucumber (Gynogenesis)** | **Cucumber/Squash (Androgenesis)** | **Carrot** | **Onion** |
|---|---|---|---|---|---|---|
| MS basal salts | Half strength MS basal salts | See Table 6 | MS basal salts | MS basal salts | Gamborg B-5 basal salts | Half strength MS basal salts |
| 0.05-0.5 mg/L Meta-Topolin | 3% sucrose | | 0.05-0.5 mg/L Meta-Topolin | 0.2 mg/L IAA | 500 mg/L L-glutamine | 3% sucrose |
| 0.01-0.1 mg/L GA3 | 0.4-4 µM IAA | | 0.01-0.1 mg/L GA3 | 0.7% agar | 100 mg/L L-serine | 0.5% agar |
| 0.5-1% plant agar | 0.6% gelrite | | 0.5-1% plant agar | 30 g/L sucrose | 2% sucrose | |
| | | | 30 g/L Sucrose | | 0.5% agar | |

### Methods

### I. Methods for Producing Di-Haploid Plants

In more general aspects, the methods of the present disclosure also provide a technology advancement, a new methodology for the induction of haploid embryogenesis and spontaneous chromosome doubling in vegetable crops from microspores and ovaries. The methods comprise pretreating a plant material, such as flower buds or anthers containing microspores, or ovaries/ovules containing the female gametophytes, under conditions which divert the microspores or female gametophyte from gametophytic development to that of embryogenic development. The pretreatment includes incubating the plant material, such as flower buds, anthers, or ovaries on an induction medium, under one or more stress conditions. The stress condition can be an elevated temperature (e.g., a heat treatment, osmotic treatment, or a combination thereof. The pretreated ovaries or the microspores, which are then released from the flower buds/anthers, are then cultured on an induction culture medium (such as Gamborg B-5 medium or Nitsch and Nitsch medium) at a standard culture temperature, such as 25°C, to induce embryogenesis. The present inventors have surprisingly discovered that pretreatment under one or more stress conditions, particularly a heat treatment applied to anthers or ovaries, can increase the efficiency of embryogenesis from the released microspores or ovaries in a genotype-independent manner.

As used herein, "heat treatment" or "heat pretreatment" or "heat shock treatment" refers to exposing the starting plant material, i.e., flower buds/anthers for microspores or ovaries/ovules for female gametophyte, to elevated temperatures for a period of time to induce embryogenesis, the formation of embryos from the microspores or female gametophyte. Heat stress treatment can trigger a cascade of molecular and cellular changes, including changes in gene expression, metabolism, and protein synthesis, that can lead to embryo formation. Heat can also affect the cell cycle of microspores or female gametophyte, promoting their division and differentiation, thereby modifying cell cycle progression.

As used herein the term "elevated temperature" refers to a temperature greater than about 25°C. In some embodiments, the elevated temperature is from about 25.1°C to about 40°C. In some embodiments, the elevated temperature is from about 25.5°C to about 40°C. In some embodiments, the elevated temperature refers to a temperature of from about 26°C to about 40°C. In some embodiments, the elevated temperature refers to a temperature of from about 26°C to about 39°C. In some embodiments, the elevated temperature refers to a temperature of from about 26°C to about 38°C. In some embodiments, the elevated temperature refers to a temperature of from about 26°C to about 37°C. In some embodiments, the elevated temperature refers to a temperature of from about 26°C to about 36°C. In some embodiments, the elevated temperature refers to a temperature of from about 26°C to about 35°C. In some embodiments, the elevated temperature refers to a temperature of from about 26°C to about 34°C. In some embodiments, the elevated temperature refers to a temperature of from about 26°C to about 33°C. In some embodiments, the elevated temperature refers to a temperature of from about 26°C to about 32°C. In some embodiments, the elevated temperature refers to a temperature of from about 26°C to about 31°C. In some embodiments, the elevated temperature refers to a temperature of from about 26°C to about 30°C. In some embodiments, the elevated temperature refers to a temperature of from about 26°C to about 29°C. In some embodiments, the elevated temperature refers to a temperature of from about 26°C to about 28°C. In some embodiments, the elevated temperature refers to a temperature of from about 26°C to about 27°C. The heat pretreatment is applied, for instance, by exposing the flower bud/anther to, at least 25.1 C, at least 25.5 °C, at least 26°C, at least 27°C, at least 28°C, at least 29°C, at least 30°C, at least 31°C, at least 32°C, at least 33°C, at least 34°C, at least 35°C, at least 36°C, at least 37°C at least 38°C, at least 39°C, or at least 40°C. Although other temperatures yield embryoids and regenerated plants, elevated temperatures produce optimal response rates compared to no pretreatment or pretreatment at temperatures outside the recited range. Microspores contained in plant material (such as isolated flower buds, inflorescences, or anthers) or isolated microspores are typically pretreated for a predetermined amount of time. In some embodiments, the plant materials are pretreated (e.g., with the heat pretreatment) for 1 to 40 days, 1 to 35 days, 1 to 30 days, 1 to 25 days, 1 to 20 days, 1 to 18 days, 1 to 15 days, or 1 to 10 days. In other embodiments, the anthers are pretreated for about 3-10 days. In some embodiments, the plant materials are treated for 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, 31 days, 32 days, 33 days, 34 days, 35 days, 36 days, 37 days, 38 days, 39 days, or 40 days. However, other amounts of time for pretreatment are within the scope of this present disclosure, as long as the plant material undergoes embryogenesis.

Similar steps are taken to produce double haploid plants through gynogenesis using ovaries/ovules to androgenesis using anthers/microspores. Like androgenesis, it is important to expose the ovary cross sections or ovaries themselves to heat pretreatment after they are placed on the induction media. However, the inventors have discovered that a prolonged heat treatment (e.g. at about 35°C) is required to kill maternal tissues, which will reduce regeneration contamination from maternal tissues (2n), and promote growth and development of double haploid embryos. In some embodiments, the ovary materials are heat pretreated for 1 to 60 days, 1 to 55 days, 1 to 50 days, 1 to 45 days, 1 to 40 days, 1 to 35 days, 1 to 30 days, 1 to 25 days, 1 to 20 days, 1 to 15 days, or 1 to 10 days. In other embodiments, the ovary materials are heat pretreated for 3-50 days. In still other embodiments, the ovary materials are heat pretreated for 10-42 days. In some embodiments, the plant materials are treated for 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, 31 days, 32 days, 33 days, 34 days, 35 days, 36 days, 37 days, 38 days, 39 days, 40 days, 41 days, 42 days, 43 days, 44 days, 45 days, 46 days, 47 days, 48 days, 49 days, 50 days, 51 days, 52 days, 53 days, 54 days, 55 days, 56 days, 57 days, 58 days, 59 days, or 60 days. However, other amounts of time for pretreatment are within the scope of this present disclosure, as long as the plant material undergoes embryogenesis.

The plant materials can be pretreated in the light or in the dark provided that the microspores or ovaries undergo embryogenesis. In some embodiments, the plant material is pretreated in the dark. As used herein, the term "dark" is defined as the absence of light in the culture environment (such as in a growth chamber without lighting, where the cultures are exposed to 0 micromole light particles as measured by a light meter), or in the cases where there is a light source (such as in a shared culture room with lighting), the culture plates are shielded from lighting by a black curtain (where the cultures are exposed to 0 micromole light particles as measured by a light meter) or by a black plastic cover (where the cultures are exposed to <0.03 micromole light particles as measured by a light meter. However, pretreatment in the light or with a light/dark cycle is also within the scope of the present disclosure, as long as the microspores will undergo embryogenesis.

The heat pretreatment in combination with the induction media resulted in a significant increase in the rate of spontaneous chromosome doubling in the regenerated pepper plants (60-90%) compared to spontaneous chromosome doubling rates that have been reported in certain prior systems. This represents an improvement of nearly 100% over existing methods. The response rate is measured as either as the number of total embryos or the number of doubled embryos per number of microspores initiated in culture.

### Di-Haploid Plants from microspores (Androgenesis)

The methods for producing di-haploid plants from microspores comprise selecting flower buds that are about 2 to about 6 mm in length from donor plants depending on the species, excising anthers from the flower buds, subjecting the anthers containing microspores to a stress condition on an induction medium, culturing or growing the microspores to allow for development into plants through embryogenesis, and to allow for spontaneous chromosome doubling, selecting regenerated di-haploid plants and growing the selected di-haploid plants to maturity and collecting seeds.

Microspores are typically competent for embryo development during their first mitosis. At this late uninucleate or early binucleate stage, the microspore has completed meiosis but has not yet fully matured into a functional pollen grain, making it receptive to external cues/stress treatment (such as temperature shock or osmotic stress) that can trigger it to switch from the gametophytic (pollen) developmental pathway to sporophytic (embryo) pathway. In one aspect, the flower buds are about 2 to about 6 mm in length, about 3 to about 5 mm in length, or about 3 mm in length, and are collected in the morning to maximize the quantity of flower buds containing microspores at a development stage amenable to embryogenesis. In another aspect, the microspores are at a late uninucleate to early binucleate stage. In certain embodiments, at least 60%, at least 70%, at least 80%, at least 90% of the microspores are at the late uninucleate to early binucleate stage. The flower buds can be collected at any time of the plant life cycle, as long as the microspores in the anthers are at the right development stage for embryogenesis. As used herein, the term "late uninucleate" stage refers to a stage where microspores contain a single laterally positioned nucleus with a large vacuole and increased number of ribosomes. At this stage, DNA in the microspores has already duplicated but is still inside one nucleus. Microspores at this stage are sensitive to the environment and most responsive to embryogenic induction. As used herein, the term "early bicellular" or "early binucleate" stage refers to a stage where microspores comprise two formed nuclei, i.e., a generative nucleus and a vegetative nucleus. Similar to microspores at the uninucleate stage, microspores at the early binucleate stage are also responsive to embryogenesis.

Methods to determine the exact stage of microspore development are known to those skilled in the art and include, but are not limited to, size of flower buds, morphology (such as size and color) of anthers based experimentally established correlation with microspore development, using fluorescent stains such as 4'-6-diamidino-2-phenylindole (DAPI) and mitramycin, or trypan blue stain, to help visualize the nuclei clearly under a microscope, and acetocarmine squashing.

### Di-Haploid Plants from Ovaries or Ovules (Gynogenesis)

The methods for producing di-haploid plants from ovaries/ovules comprise selecting unfertilized ovaries from about six hours to about 3 days before anthesis from donor plants, slicing the ovaries into about 2 mm cross sections, subjecting the slices to a stress condition on an induction medium, culturing the ovary slices to allow development into plants through embryogenesis and to allow for spontaneous chromosome doubling, selecting regenerated di-haploid plants, and growing the selected di-haploid plants to maturity and collecting seeds.

Gynogenesis is the induction of an unfertilized ovule or ovary to develop into an embryo, essentially bypassing the need for fertilization by a male gamete. It involves culturing unfertilized ovules or ovaries *in vitro* on a nutrient medium. Plant growth regulators are added to the culture medium to stimulate the development of the unfertilized egg cell into an embryo. Gynogenesis provides another pathway to produce haploid or double haploid plant, which is also valuable in plant breeding for faster development of homozygous lines.

For successful haploid and double haploid plant production through gynogenesis, the development stage of the ovule or embryo sac (female gametophyte) at the time of ovule/ovary culture is critical. In some embodiments, the ovaries are selected from donor plants about one month after the emergence of the second female flower, when the corolla exhibited a yellow-green hue, and most ovaries contained nearly mature embryonic sacs, making them ideal explants for inducing *in vitro* gynogenesis. In other embodiments, the unfertilized ovaries were collected from about six hours to about 3 days, from about six hours to about 2 days, from about six hours to about 1 day, or from about six hours to about 12 hours before anthesis.

Methods for determining the stage of plant embryo sac development are known in the art, including, but are not limited to microscopic observations (such as histological studies, paraffin sectioning, fluorescence staining, or scanning electron microscopy), and molecular techniques (such as quantitative reverse transcription polymerase chain reaction, transcriptome analysis, proteomics, RNA sequencing).

### Chromosome Doubling

Microspore-derived or ovary/ovule-derived haploid embryos can be converted to doubled haploids by chromosome-doubling agents and/or through spontaneous doubling. Chromosome-doubling agents are known in the art and include colchicine, oryzalin, trifluralin, amiprophos-methyl (APM), pronamide, etc. These agents generally work by disrupting the microtubule system, which is essential for proper chromosome separation during cell division. While useful in producing polyploid plants with desirable traits in plant breeding, chromosome doubling agents, especially colchicine which is most widely used, are toxic to both plants and humans. Colchicine can cause various adverse effects, including development problems in plants and potential poisoning in humans if handled improperly. Furthermore, colchicine treatment can lead to abnormalities in plant growth, such as delayed flowering, malformed flowers, and changes in leaf color. It can also induce chromosome instability in some cases.

Spontaneous chromosome doubling in haploid plants, also known as spontaneous haploid genome doubling, occurs when a haploid cell, derived from pollen or egg cells, naturally or grown under *in vitro* culture conditions duplicates its chromosomes, resulting in a fertile doubled haploid (DH) plant with a homozygous genotype. This process bypasses the need for chemical treatments like colchicine to induce chromosome doubling. The exact mechanisms of spontaneous chromosome doubling are not fully understood, but it is thought to occur through processes like nuclear fusion (involving joining of two nuclei, potentially leading to a diploid cell), endoreduplication (referring to DNA replication without cell division, leading to a doubling of the chromosome number), or other mechanisms during early embryogenic development.

The methods of the present disclosure represent a major technology advancement in producing double haploid vegetable crop plants. The combination of selecting anther or ovary material at the right stage of development, heat stress pretreatment and induction media resulted in high rates of double haploid plant production, thereby eliminating the need for toxic chemical treatment. Molecular analysis reveals that spontaneous chromosome doubling occurs at an early stage of the induced embryo development.

As used herein, a "haploid plant" refers to a plant that contains only a single set of chromosomes in each cell. Haploid plants are created from male or female gametes or gamete-like cells, which don't go through fertilization. A "double haploid plant" or "doubled haploid plant" refers to a plant that is produced by doubling the chromosome number of each cell by chemical means or spontaneous doubling, resulting in a diploid plant that is homozygous at every single locus (the specific location on a chromosome where a particular gene is found). Because double haploid plants have homozygous, or pure-breeding, genetic makeup, they can be used to rapidly develop inbred lines in plant breeding. As used herein, a "di-haploid" or "di-H" or "diH" or "Di-H" or "DiH" plant is a plant with doubled set of chromosomes derived from a haploid plant, achieved through spontaneous chromosome-doubling without the need to use harmful chemical doubling agent such as colchicine.

Di-haploid plants produced by the methods of the present disclosure are characterized by complete homozygosity at substantially all genetic loci, absence of chimeric tissues that typically result from chemical chromosome doubling agents, and uniform diploid ploidy level across substantially all cells (e.g., at least 95% of cells are diploid as determined by flow cytometry). These characteristics distinguish di-haploid plants of the present disclosure from chemically doubled haploid plants, which frequently exhibit mixed ploidy, chimeric tissues, and growth abnormalities.

As used herein, when referring to chromosome count, "doubling" refers to increasing the chromosome number by a factor of two. For example, a haploid nuclear genome comprising 12 chromosomes becomes a diploid nuclear genome comprising 24 chromosomes when doubled. As another example, a diploid nuclear genome comprising 24 chromosomes becomes a tetraploid nuclear genome comprising 48 chromosomes when doubled.

Microspores and ovaries cultured under the suitable conditions as described in the present disclosure generally go through embryogenesis and develop into globular embryos. These globular embryos are then cultured under conditions effective for them to undergo histodifferentiation, a process where a globular stage embryo develops into a mature embryo with distinct tissues and organs, including cotyledons, shoot and root meristem, and a hypocotyl. The density of the anthers/microspores placed on the induction medium plays an important role in the success of androgenesis and regeneration of double haploid plants. The concentration of microspores in the induction medium directly influences the physiological and biochemical environment necessary for embryogenic induction. At optimal densities, microspores can engage in effective cell-to-cell signaling and secrete growth-promoting factors that support the switch from the gametophytic to the sporophytic pathway. If the density is too low, the microspores may lack sufficient stimulation for embryogenesis, leading to low induction rates. Conversely, excessively high densities can result in the accumulation of toxic metabolites, nutrient depletion, and cellular stress, all of which impair viability and embryo development. Therefore, optimizing microspore density is essential for maximizing the efficiency, consistency, and scalability of androgenic regeneration systems in crop breeding programs. In one embodiment, the method is carried out on a microspore population comprising about 24,000 microspores (15 anthers) per induction plate, about 19,200 microspores (12 anthers) per induction plate, about 16,000 microspores (10 anthers) per induction plate, about 11,200 microspores (7 anthers) per induction plate, about 8,000 (5 anthers) microspores per induction plate, or about 3,200 microspores (2 anthers) per induction plate.

### Selection of Di-Haploid Plants

Double haploid plants, being homozygous, can be identified and selected through various methods known in the art, including visual analysis, molecular markers, and cytological techniques.

Haploid plants often exhibit morphological differences compared to their diploid counterparts, such as shorter stature, smaller internodes, and fewer leaves.

Various cytological methods can be used to determine the ploidy level of the regenerated plants, such as flow cytometry and karyotype analysis. Flow cytometry provides a rapid and efficient method for distinguishing between diploid (2n) and haploid (n) plant cells (Eeckhaut et al, 2005) by quantifying their DNA content. Karyotype analysis can be used to determine the ploidy level of a plant by examining its chromosomes. It involves staining and arranging chromosomes during metaphase, a phase of cell division where they are most condensed and visible. By counting the number of chromosomes, the regenerated plants' ploidy levels can be determined.

Various molecular methods can be used to distinguish between haploid and double haploid plants, including but not limited to, single nucleotide polymorphism (SNP) genotyping, chromosomal microarray analysis, fluorescence in situ hybridization (FISH), next-generation sequencing (NGS), microsatellite analysis (SSR) or other molecular biology techniques known in the art.

The term "genotype" refers to the genetic makeup of an individual (or group of individuals) at one or more genetic loci, as contrasted with the observable trait (the phenotype). Genotype is defined by the allele(s) of one or more known loci that the individual has inherited from its parents. The term genotype can be used to refer to an individual's genetic constitution at a single locus, at multiple loci. Or, more generally, the term genotype can be used to refer to an individual's genetic make-up for all the genes in its genome, or its entire genetic makeup, which determines the individual plant's traits or characteristics or phenotypes. The terms "phenotype," or "phenotypic trait" or "trait" refers to one or more traits of an organism. The phenotype can be observable to the naked eye, or by any other means of evaluation known in the art, e.g., microscopy, biochemical analysis, genomic analysis, an assay for a particular disease resistance, etc. In some cases, a phenotype is directly controlled by a single gene or genetic locus, i.e., a "single gene trait." In other cases, a phenotype is the result of the expression of several genes and their interaction with environments.

The term "genotyping" refers to a laboratory process that analyzes an individual's DNA sequence to identify specific differences or variations, or alleles at specific locations in the genome, helping determine their genetic makeup, or genotype. Genotyping methods are known in the art, including, but not limited to, DNA sequencing (such as Sanger Sequencing, Next Generation Sequencing (NGS), Genotyping by Sequencing (GBS) and Targeted NGS), DNA Microarrays, PCR-Based Genotyping (such as rhAmp SNP Genotyping, High-Resolution Melting (HRM), Digital PCR), Mass Array SNP Genotyping, Genotyping using Hybridization, Genetic Engineering, and Genomic Prediction.

As used herein, the term "low coverage sequencing" for double haploid (DH) or di-haploid plants refers to the generation of genomic sequence data at a depth of less than 1.0X, which is subsequently utilized to reconstruct the hybrid genome by mapping sparse sequence reads to established parental haplotypes, leveraging the inherent homozygosity of the DH lines to ensure high-fidelity imputation of missing loci (Weller, et al, 2021; Xu, et al, 2016)

The term "marker panel" refers to a collection of DNA markers (such as SNPs) that are used to identify genetic variations or traits in a sample. These markers act as "landmarks" on the genome, allowing researchers to track inheritance patterns and identify genes associated with specific traits. Marker panels are very useful in determining the genetic makeup (genotype) of individuals or populations, creating genetic maps, identifying genes or regions of the genome associated with specific traits or diseases, and assisting in selecting individuals with desirable traits for breeding programs.

### II. Methods for Accelerated Selection of New Commercial Hybrid Vegetable Varieties

The present disclosure provides a significant improvement over prior art for producing double haploid plant cells, plants and seeds in vegetable species, such as in the *Solanaceae* and *Cucurbitaceae* families, with high efficiency, shorter time duration, and in a genotype-independent manner. Key improvements include: 1) Development of microspore culture systems through embryogenesis in a genotype independent manner. Many vegetable varieties previously considered as "recalcitrant" have been successfully tested to produce many healthy di-haploid embryos that were subsequently germinated into viable plants and produced seeds. 2) The surprisingly significant increase in the rate of spontaneous chromosome doubling in the regenerated plants (60-90%) compared to spontaneous chromosome doubling rates that have been reported in certain prior systems. This represents an improvement of nearly 100% over existing methods. Such a substantial increase in spontaneous chromosome doubling rate is a major advancement in doubled haploid (DH) technology for *Solanaceae* crops. This improvement can significantly reduce the need for artificial chromosome doubling treatments (e.g., colchicine), which are often labor-intensive and potentially hazardous to human, and can be harmful to plant tissues, and produce undesirable chimera plants.

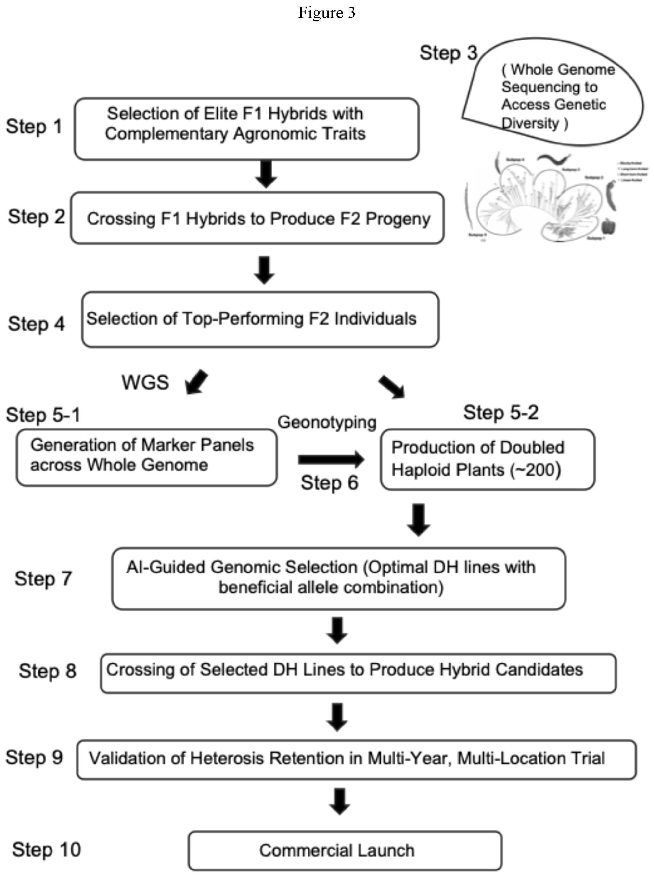

Traditionally, vegetable breeders recycle inbred lines in their breeding programs to develop new F1 hybrids. However, this method is limited by genetic constraints, and many crosses rely on phenotypic observations of parental lines, introducing subjective bias or human bias. Figure 3 provides a flowchart demonstrating a novel approach combining four-way crossing with the di-haploid method of the present disclosure in vegetable breeding for accelerated selection of new commercial hybrids with higher heterosis compared to the two original parental commercial hybrids using pepper (*Capsicum annuum*) as an example. In the approach of the present disclosure, two elite F1 hybrids with complementary agronomic traits, such as yield, fruit quality, disease resistance, and stress tolerance, are selected (Step 1, FIG. 3) and crossed to generate a diverse F2 population (Step 2, FIG. 3). Whole genome sequencing is applied to identify F1 hybrids with broad genetic backgrounds, maximizing the potential for heterotic recombination in the progeny (Step 3, FIG. 3).

The resulting F2 population, containing novel recombinants, is evaluated for key performance traits (Step 4, FIG 3). The best- or top-performers, exhibiting superior heterosis compared to both F1 parents, are selected and sequenced for genomic selection (Step 5-1, FIG. 3). These top-performing F2 individuals are subjected to double haploid production using the methods of the present disclosure to generate approximately 200 DH/DiH plants (Step 5-2, FIG. 3). These plants are then genotyped using the heterozygous markers identified from the selected F2 genomic analysis by whole genome sequencing (Step 6, FIG. 3).

Using AI-assisted genomic selection (Step 7, FIG. 3), DH lines with optimal genetic combinations are crossed to produce hybrid candidates (Step 8, FIG. 3). These candidates undergo multi-location, multi-year trials to confirm heterosis retention (Step 9, Fig. 3). The best-performing hybrids are then advanced for commercial release (Step 10, FIG. 3).

Selection refers to identifying di-haploid parental lines to cross to test potential hybrids. As the number of potential hybrids for a given crossing block is equal to N choose 2, for instance 100 di-haploids can be crossed in 4,950 different ways, a methodology to identify the crosses most likely to produce a desirable outcome is desired. Essentially, the AI algorithm is used to reduce the crossing blocks that enter testing to a manageable number that are likely to perform significantly better than a randomly selected panel.

The most straightforward approach is to select crosses based on maximum genetic distance using markers or sequencing data (Flint-Garcia et al., 2009; Tomkowiak et al., 2020). Each potential cross is scored based on the following equation: ${D}_{x , y} = \frac{1}{pn} {\sum }_{i = 1}^{n} {\sum }_{j = 1}^{m} \sqrt{{\left({A}_{i , j , x}-{A}_{i , j , y}\right)}^{2}}$

Where *x* and *y* are the two candidate parents under consideration, *D* is the fraction of alleles that differ between them, p is the ploidy (*i.e.* a diploid would have a ploidy of 2), n is the total number of markers, m is the total number of alleles at marker *I*, and *A* the allele count for a given parent at marker *i* for allele sequence *j.* The underlying concept is that hybrid vigor often correlates with genetic distance, and as such, maximizing genetic distance is one way to identify optimal pairings. An alternative way to calculate this value is to subtract the Jaccard index for the two samples from one.

One approach to refining the measurement of genetic distance is to assign a weight to each marker. This weight can be based on training data from a previous population in a genomic selection model, or it can be based on prior knowledge of the genome. The most common hypothesis for heterosis states that it is achieved by minimizing genetic load by ensuring as few functional variants are homozygous as possible (Charlesworth & Willis, 2009; Yang et al., 2017; Yao et al., 2013). As such, regions can be weighed both by the number of genes in them and the number of functional variants that are heterozygous for a given cross in those genes. The following equation is applied: ${S}_{x , y} = {\sum }_{i = 1}^{n} {W}_{i , i - 1} \left({H}_{i , x , y}+{H}_{i - 1 , x , y}\right)$

Where *x* and *y* are the two candidate parent lines under consideration, *S* is the score, *n* is the total number of markers, *W* is the weight of the region between two markers, and *H* is the likelihood that the offspring of the candidate parents will be heterozygous at marker *i.* While *W* is derived from whole genome sequencing data for the background line(s) of the parents, the specifics can vary. In the case of this example, it is an amalgamation of the total distance, the number of genes, and the number and severity of any identified functional variants. It is not solely the number of functional variants since these may vary in impact based on the nature of the variant and the importance of the gene, not all functional variants may be accurately identified, and not all genes and other functional elements such as non-coding RNAs may be accurately identified. We do, however, assume that gene density and the genetic variation within the region correlate with the actual contribution of the region to hybrid vigor.

As used herein, the phrase "AI-assisted genomic selection" refers to the utilization of one or more AI algorithms to analyze vast genomic datasets of plant DNA and phenotype information. This allows for more accurate prediction of plant performance, resistance to stress, and yield, leading to breeding values, and optimizing selection strategies, ultimately accelerating and making the development of improved crop varieties more efficient. As used herein, "genomic selection (GS)" refers to a breeding strategy that utilizes genome-wide markers (like SNPs) to predict the breeding value of individuals, rather than relying solely on phenotypic data. It leverages the power of genomics to identify individuals with superior genetic potential, even before they are phenotyped. This allows for faster and more accurate selection, increasing genetic gain per breeding cycle.

AI assists in genomic selection through 1) data analysis: AI algorithms can efficiently analyze large and complex genomic datasets, identifying patterns and relationships that traditional methods might miss; 2) prediction models: AI can be used to develop and refine genomic prediction models, improving the accuracy of breeding value predictions; 3) optimized selection: AI can help optimize selection strategies, such as identifying the best mating or crossing combinations or selecting the most promising individuals for breeding programs, maximizing the genetic diversity and desired traits in the offsprings. AI-assisted genomic selection has been described in the literature, for example by Hamazaki and Iwata (2024).

Benefits of AI-assisted genomic selection include 1) increased accuracy: AI-powered prediction models can lead to more accurate breeding value predictions, resulting in better selection decisions; 2) faster breeding cycles: by predicting breeding values based on genomic data, genomic selection allows for faster selection and shorter breeding cycles; 3) reduced cost: genomic selection can reduce the need for extensive phenotypic data collection, leading to cost savings; 4) improved genetic gain: by selecting individuals with superior genetic potential, genomic selection can lead to greater genetic gains per breeding cycle; and/or 5) targeted breeding: AI can help identify specific genes or genomic regions associated with desired traits, allowing to targeted breeding programs.

Compared to traditional forward genetics methods, the methods described herein capture novel recombination events and fix beneficial alleles through natural meiotic recombination and DH technology. Additionally, using DH lines enables the rapid stabilization of superior parental lines, significantly accelerating the breeding process. The present disclosure addresses the limitation of traditional breeding by enabling rapid production of homozygous lines with novel trait combinations from selected offspring of the four-way crosses. It allows breeders to swiftly evaluate the potential of diverse genetic combinations, which significantly reduces the timeline to 1-2 years compared to the 5-7 years required by traditional selfing (i.e., sexual crossing of identical genotypes) breeding approach.

As used herein, an "allele" refers to one of two or more alternative versions of a DNA sequence (a single base or a segment of bases) at a given genomic location, or locus or gene, with individuals inheriting two alleles for each locus or gene, one from each parent at the same position (or locus), that can lead to variations in traits. For example, a first allele can occur on one chromosome, while a second allele occurs on the homologous position of a second chromosome, e.g., as occurs for different chromosomes of a heterozygous individual, or between different homozygous or heterozygous individuals in a population. A favorable allele is an allele at a particular locus that confers, or contributes to, an agronomically desirable phenotype, or alternatively, is an allele that allows the identification of susceptible plants that can be removed from a breeding program or planting. A favorable allele of a marker is a marker allele that segregates with the favorable phenotype, or alternatively, segregates with susceptible plant phenotype, therefore providing the benefit of identifying disease prone plants. A favorable allelic form of a chromosome site or segment is a chromosome site or segment that includes a nucleotide sequence that contributes to superior agronomic performance at one or more genetic loci physically located on the chromosome interval. An allele positively correlates with a trait when it is linked to it and when the presence of the allele indicates that the desired trait or trait form will occur in a plant comprising the allele. An allele negatively correlates with a trait when it is linked to it and when the presence of the allele indicates that a desired trait or trait form will not occur in a plant comprising the allele.

As used herein, "cross" or "crossed" means to produce progeny through fertilization, either by "cross pollination", where pollen, containing the male reproductive cell (sperm), is transferred from the anthers of a flower on one plant to the stigma of a flower on another plant of the same species, leading to increased genetic diversity and plant adaptability; or by "selfing" or "self-pollination", where a plant's ovules are fertilized by pollen from the same plant, to maintain the purity of a variety, to develop true breeding lines and to increase the homozygosity.

The term "variety" or "cultivar" refers to a group of plants within a species that share a distinct set of characteristics. These characteristics are often selected and maintained by humans through selective breeding or propagation.

As used herein, the term "heterosis", also known as hybrid vigor, refers to the phenomenon where hybrid offspring exhibit superior traits or enhanced biological function, such as increased yield, growth rate, and disease resistance, compared to their parents, often due to increased genetic diversity. Heterosis can result from complex genetic interactions, including dominance, overdominance, and epistasis. The mixing of diverse parental genomes can lead to the masking of deleterious recessive alleles and the expression of beneficial alleles. Heterosis is a key factor in crop breeding, allowing for the development of improved varieties with higher yield and desirable traits. "Heterosis retention" refers to maintaining the benefits of hybrid vigor over multiple generations through strategic breeding programs. As used herein, "top performing F2 plants" refers to F2 progeny plants demonstrating one or more desirable key agronomic and/or market-driven traits or phenotypes when compared to their F1 hybrid parents. The desirable traits or phenotypes include, but are not limited to, disease resistance or tolerance; early flowering time; synchronous flower set, with no observable flower abortion, contributing to reliable fruit initiation and increased fruit set; early fruit setting and high fruit load; uniform, market-preferred fruit types with high visual appeal; consistent fruit shape and color, and firm texture, indicative of strong shelf-life potential fruit shape; fruit color; and increased fruit size and average fruit weight, with select crosses reaching commercial thresholds for fresh and processing segments; and increased yield. As used herein, desirable "fruit quality" includes uniform, market-preferred fruit types with high visual appeal; consistent fruit shape and color, and firm texture, indicative of strong shelf-life potential fruit shape; fruit color; and increased fruit size and average fruit weight. The combination of strong field vigor, reproductive efficiency, and superior fruit quality make these F₂ selections promising candidates for potential hybrid development.

The improved breeding methods of the present disclosure combine the power of AI-assisted genomic selection with the high frequency double haploid production to allow for the capture of novel recombination events and fixation of beneficial alleles through natural meiotic recombination and double haploid technology at a scale that is otherwise impossible. The present disclosure addresses the limitation of traditional breeding by enabling rapid production of homozygous lines with novel trait combinations from selected offspring of the parental hybrid by hybrid crosses and allows breeder to rapidly evaluate the potential of diverse genetic combinations, which significantly reduced the timeline to 1-2 years compared to the 5-7 years required by traditional selfing breeding approach.

Each step of the accelerated hybrid selection method is enabled by the teachings of the present disclosure and by techniques known to persons of ordinary skill in the art. Step d), producing di-haploid plants from selected F2 plants, is enabled by the detailed protocols and working examples disclosed herein (Examples 1, 4-9), which demonstrate genotype-independent di-haploid production across multiple species. Steps a) through c) (parental selection, F2 generation, and selection with whole genome sequencing) employ standard breeding and genomic techniques available to any plant breeder equipped with modern sequencing capabilities. Step e) (genotyping di-haploid plants using heterozygous markers) is demonstrated in Examples 1 and 5-6, which provide detailed SNP genotyping protocols using Fluidigm^{®} SNP Type^{™} assays. Step f) (AI-assisted genomic selection) is described herein with specific mathematical frameworks (genetic distance calculation and weighted marker scoring equations) that provide a person of ordinary skill in the art with sufficient guidance to implement such selection. Steps g) and h) (crossing di-haploid lines and field trial validation) employ conventional hybrid breeding techniques that are routinely practiced in the art. A person of ordinary skill in the art, equipped with the di-haploid production methods, genotyping protocols, and AI-assisted genomic selection frameworks disclosed herein, together with standard plant breeding practices, would be able to practice the complete accelerated hybrid selection method without undue experimentation.

The above description is given by way of example, and not limitation. Given the above disclosure, one skilled in the art could devise variations that are within the scope and spirit of the invention disclosed herein. Further, the various features of the embodiments disclosed herein can be used alone, or in varying combinations with each other and are not intended to be limited to the specific combination described herein. Thus, the scope of the claims is not to be limited by the illustrated embodiments.

For reasons of completeness, various aspects of the disclosure are set out in the following numbered embodiments:
Numbered embodiment 1. A method for producing a di-haploid plant from microspores, the method comprising:
   a) selecting one or more plant flower buds having a length of about 2 to about 6 mm;
   b) excising one or more anthers containing microspores from the flower buds;
   c) subjecting the anthers containing microspores to one or more stress conditions;
   d) culturing or growing anthers containing microspores on an induction medium to allow: (i) the microspores to develop into plants; and (ii) for spontaneous chromosome doubling, wherein the chromosome doubling results in di-haploid plants;
   e) selecting the di-haploid plants; and
   f) growing the selected di-haploid plants to maturity and collecting seed.
Numbered embodiment 2. The method of Numbered embodiment 1, wherein the induction medium comprises at least: a) one or more ammonium salts; b) calcium chloride; c) boric acid; d) cupric sulfate; e) magnesium sulfate; f) manganese sulfate; g) molybdic acid (sodium salt); h) potassium nitrate; i) potassium phosphate or sodium phosphate; j) zinc sulfate; k) myo-inositol; l) nicotinic acid; m) pyridoxine HCl; n) thiamine HCl; and o) one or more plant growth regulators selected from the group consisting of: (i) 0.1-1 mg/L IAA and 0.05-2 mg/L zeatin; (ii) 0.1 mg/L 2,4-D, 0.88 mg/L IAA, and 0.55 mg/L zeatin; (iii) 0.02-5 µM 2,4-D; (iv) 5 mg/L kinetin and 5 mg/L 2,4-D; and (v) 0.01-0.5 mg/L thidiazuron (TDZ) and 1-10 mg/L zeatin..
Numbered embodiment 3. The method of Numbered embodiment 2, wherein the ammonium salt is ammonium nitrate or ammonium sulfate.
Numbered embodiment 4. The method of any of Numbered embodiments 1-3, wherein the microspores are at a late uninucleate to early binucleate development stage.
Numbered embodiment 5. The method of any of Numbered embodiments 1-4, wherein the stress condition is a heat treatment.
Numbered embodiment 6. The method of Numbered embodiment 5, wherein the heat treatment is carried out at about 26-40°C for 1-40 days in the dark.
Numbered embodiment 7. The method of Numbered embodiment 6, wherein the heat treatment is carried out at about 27-37°C for 3-10 days in the dark.
Numbered embodiment 8. The method of any of Numbered embodiments 1-7, wherein the stress condition is a combination of heat and osmotic treatment.
Numbered embodiment 9. The method of Numbered embodiment 8, wherein the heat treatment is carried out at 26-40°C for 1-40 days in the dark, and the osmotic treatment comprises adding about 100 to about 120 g/L sucrose to the induction medium or supplying about 100 to about 120 g/L of sucrose to the induction medium.
Numbered embodiment 10. The method of any of Numbered embodiments 1-9, wherein the selecting of di-haploid plants comprises using flow cytometry.
Numbered embodiment 11. The method of any of Numbered embodiments 1-9, wherein the selecting of di-haploid plants uses genotyping.
Numbered embodiment 12. The method of any of Numbered embodiments 1-11, wherein the di-haploid plant is a *Solanaceae* crop plant, a *Cucurbitaceae* crop plant, or an *Apiaceae* crop plant.
Numbered embodiment 13. The method of Numbered embodiment 12, wherein the *Solanaceae* crop plant is a tomato (*Lycopersicon)* plant, a pepper (*Capsicum)* plant, or an eggplant (*Solanum*) plant.
Numbered embodiment 14. The method of Numbered embodiment 12, wherein the *Cucurbitaceae* crop plant is a zucchini *(Cucurbita)* crop plant, a pumpkin *(Cucurbita)* crop plant, or a melon *(Cucumis)* crop plant.
Numbered embodiment 15. The method of Numbered embodiment 12, wherein the *Apiaceae* crop plant is a carrot plant.
Numbered embodiment 16. A di-haploid plant, plant part, plant seed, or plant embryo produced by the method of any of Numbered embodiments 1-15, wherein the di-haploid plant is homozygous at substantially all genetic loci and is free of chimeric tissues.
Numbered embodiment 17. A medium composition for inducing embryogenesis and spontaneous chromosome doubling of regenerated plants from cultured microspores or ovaries, the medium comprising: at least a) one or more ammonium salt; b) calcium chloride; c) boric acid; d) cupric sulfate; e) magnesium sulfate; f) manganese sulfate, g) molybdic acid (sodium salt); h) potassium nitrate; i) potassium phosphate or sodium phosphate; j) zinc sulfate; k) myo-inositol; l) nicotinic acid; m) pyridoxine HCl; n) thiamine HCl; and o) one or more plant growth regulators selected from the group consisting of: (i) 0.1-1 mg/L IAA and 0.05-2 mg/L zeatin; (ii) 0.1 mg/L 2,4-D, 0.88 mg/L IAA, and 0.55 mg/L zeatin; (iii) 0.02-5 µM 2,4-D; (iv) 5 mg/L kinetin and 5 mg/L 2,4-D; and (v) 0.01-0.5 mg/L thidiazuron (TDZ); and p) 1-10 mg/L zeatin.
Numbered embodiment 18. The medium composition of Numbered embodiment 17, wherein the ammonium salt is ammonium nitrate or ammonium sulfate.
Numbered embodiment 19. The medium composition of Numbered embodiment 17 or Numbered embodiment 18, wherein the medium is a liquid medium.
Numbered embodiment 20. The medium composition of Numbered embodiment 17 or Numbered embodiment 18, wherein the medium is a semi-solid medium.
Numbered embodiment 21. The medium composition of any of Numbered embodiments 17-20, wherein the medium comprises a bottom semi-solid medium layer and an upper liquid medium layer.
Numbered embodiment 22. A method for producing a di-haploid plant from ovaries or ovules, the method comprising:
   a) selecting and collecting unfertilized ovaries about six hours before anthesis;
   b) slicing the ovaries into about 1-5 mm cross sections;
   c) subjecting the slices to one or more stress conditions;
   d) culturing or growing ovary slices on an induction medium to allow: (i) development into plants; and (ii) for spontaneous chromosome doubling, wherein the chromosome doubling results in di-haploid plants;
   e) selecting di-haploid plants; and
   f) growing the selected di-haploid plants to maturity and collecting seed.
Numbered embodiment 23. The method of Numbered embodiment 22, wherein the induction medium comprises at least a) one or more ammonium salt; b) calcium chloride; c) boric acid; d) cupric sulfate; e) magnesium sulfate; f) manganese sulfate, g) molybdic acid (sodium salt); h) potassium nitrate; i) potassium phosphate or sodium phosphate; j) zinc sulfate; k) myo-inositol; l) nicotinic acid; m) pyridoxine HCl; n) thiamine HCl; o) (v) 0.01-0.5 mg/L thidiazuron (TDZ).
Numbered embodiment 24. The method of Numbered embodiment 23, wherein the ammonium salt is ammonium nitrate or ammonium sulfate.
Numbered embodiment 25. The method of any of Numbered embodiments 22-24, wherein the stress condition is a heat treatment.
Numbered embodiment 26. The method of Numbered embodiment 25, wherein the heat treatment is carried out at 26-40°C for 1-60 days in the dark.
Numbered embodiment 27. The method of Numbered embodiment 26, wherein the heat treatment is carried out at 27-37°C for 1-42 days in the dark.
Numbered embodiment 28. The method of any of Numbered embodiments 22-27, wherein the selecting of regenerated di-haploid plants is using flow cytometry.
Numbered embodiment 29. The method of any of Numbered embodiments 22-27, wherein the selecting of regenerated di-haploid plants is using genotyping.
Numbered embodiment 30. The method of any of Numbered embodiments 22-29, wherein the di-haploid plant is a *Cucurbitaceae* crop plant, or an *Amaryllidaceae* crop plant.
Numbered embodiment 31. The method of Numbered embodiment 30, wherein the *Cucurbitaceae* crop plant is a zucchini *(Cucurbita)* crop plant, a cucumber *(Cucumis)* crop plant, a pumpkin *(Cucurbita)* crop plant, or a melon *(Cucumis)* crop plant.
Numbered embodiment 32. The method of Numbered embodiment 30, wherein the *Amaryllidaceae* crop plant is an onion (*Allium*) crop plant.
Numbered embodiment 33. A di-haploid plant, plant part, plant bulb, plant seed, or plant embryo produced by the method of any of Numbered embodiments 22-32.
Numbered embodiment 34. A method for accelerated selection of new commercial hybrid vegetable varieties, the method comprising:
   a) selecting elite F1 hybrid plants with broad genetic backgrounds and/or complementary agronomic traits;
   b) crossing the selected F1 hybrid plants to produce F2 populations;
   c) selecting top-performing F2 plants and performing whole genome sequencing to generate marker panel;
   d) producing di-haploid plants from the selected F2 plants using the methods of Numbered embodiments 1-15 or Numbered embodiments 22-32;
   e) subjecting the produced di-haploid plants to genotyping using heterozygous markers generated from the marker panel;
   f) selecting di-haploid lines with beneficial allele combinations using AI-assisted genomic selection;
   g) crossing the selected di-haploid lines to produce hybrid candidates; and
   h) validating heterosis performance in the hybrid candidates through field trials.
Numbered embodiment 35. The method of Numbered embodiment 34 wherein the selecting elite F1 hybrid plants in step a) involves the use of whole genome sequencing.
Numbered embodiment 36. The method of Numbered embodiment 34 or Numbered embodiment 35, wherein the agronomic traits are selected from the group consisting of: yield, fruit quality, disease resistance, and stress tolerance, or any combination thereof.
Numbered embodiment 37. A method for producing a di-haploid *Solanaceae* plant from microspores, the method comprising:
   a) selecting one or more *Solanaceae* plant flower buds having a length of about 2 to about 6 mm;
   b) excising one or more anthers containing microspores from the flower buds;
   c) subjecting the anthers containing microspores to one or more stress conditions selected from the group consisting of heat treatment, osmotic treatment, or a combination of heat and osmotic treatments;
   d) culturing or growing anthers containing microspores on an induction medium to allow: (i) the microspores to develop into plants; and (ii) for spontaneous chromosome doubling, wherein the chromosome doubling results in di-haploid plants;
   e) selecting the di-haploid *Solanaceae* plants; and
   f) growing the selected di-haploid *Solanaceae* plants to maturity and collecting seed.
Numbered embodiment 38. The method of Numbered embodiment 37, wherein the induction medium comprises at least: a) one or more ammonium salts; b) calcium chloride; c) boric acid; d) cupric sulfate; e) magnesium sulfate; f) manganese sulfate; g) molybdic acid (sodium salt); h) potassium nitrate; i) potassium phosphate or sodium phosphate; j) zinc sulfate; k) myo-inositol; 1) nicotinic acid; m) pyridoxine HCl; n) thiamine HCl; and o) one or more plant growth regulators selected from the group consisting of: (i) 0.1-1 mg/L IAA and 0.05-2 mg/L zeatin; (ii) 0.1 mg/L 2,4-D, 0.88 mg/L IAA, and 0.55 mg/L zeatin; (iii) 0.02-5 µM 2,4-D; (iv) 5 mg/L kinetin and 5 mg/L 2,4-D; and (v) 0.01-0.5 mg/L thidiazuron (TDZ) and 1-10 mg/L zeatin..
Numbered embodiment 39. The method of Numbered embodiment 38, wherein the ammonium salt is ammonium nitrate or ammonium sulfate.
Numbered embodiment 40. The method of any of Numbered embodiments 37-39, wherein the microspores are at a late uninucleate to early binucleate development stage.
Numbered embodiment 41. The method of any of Numbered embodiments 37-40, wherein the heat treatment is carried out at about 26-40°C for 1-40 days in the dark.
Numbered embodiment 42. The method of any of Numbered embodiments 37-40, wherein the heat treatment is carried out at about 27-37°C for 3-10 days in the dark.
Numbered embodiment 43. The method of any of Numbered embodiments 37-42, wherein the osmotic treatment comprises supplying about 100 to about 120 g/L of sucrose to the induction medium.
Numbered embodiment 44. The method of any of Numbered embodiments 37-43, wherein the selecting of di-haploid *Solanaceae* plants comprises using flow cytometry or genotyping.
Numbered embodiment 45. The method of any of Numbered embodiments 37-44, wherein the *Solanaceae* plant is selected from the group consisting of tomato, pepper and eggplant.
Numbered embodiment 46. A di-haploid *Solanaceae* plant, plant part, plant seed, or plant embryo produced by the method of any of Numbered embodiments 37-45, wherein the di-haploid plant is homozygous at substantially all genetic loci and is free of chimeric tissues.
Numbered embodiment 47. The method of any of Numbered embodiments 37-39, wherein the medium is a liquid medium, a semi-solid medium, or a combination comprising a semi-solid layer and a liquid layer.
Numbered embodiment 48. A method for producing a di-haploid *Cucurbitaceae* plant from ovaries or ovules, the method comprising:
   a) selecting and collecting unfertilized ovaries from a *Cucurbitaceae* plant about six hours before anthesis;
   b) slicing the ovaries into about 1-5 mm cross sections;
   c) subjecting the slices to one or more stress conditions selected from the group consisting of heat treatment, osmotic treatment, or a combination of heat and osmotic treatments;
   d) culturing or growing ovary slices on an induction medium to allow: (i) development into plants; and (ii) for spontaneous chromosome doubling, wherein the chromosome doubling results in di-haploid *Cucurbitaceae* plants;
   e) selecting the di-haploid *Cucurbitaceae* plants; and
   f) growing the selected di-haploid *Cucurbitaceae* plants to maturity and collecting seed.
Numbered embodiment 49. The method of Numbered embodiment 48, wherein the induction medium comprises the components of Numbered embodiment 38.
Numbered embodiment 50. The method of Numbered embodiment 49, wherein the ammonium salt is ammonium nitrate or ammonium sulfate.
Numbered embodiment 51. The method of any of Numbered embodiments 48-50, wherein the heat treatment.is carried out at about 26-40°C for 1-60 days in the dark.
Numbered embodiment 52. The method of any of Numbered embodiments 48-50, wherein the heat treatment is carried out at 27-37°C for 1-42 days in the dark.
Numbered embodiment 53. The method of any of Numbered embodiments 48-52, wherein the selecting comprises using flow cytometry, genotyping, or low coverage sequencing.
Numbered embodiment 54. The method of any of Numbered embodiments 48-53, wherein the *Cucurbitaceae* plant is zucchini, cucumber, pumpkin, and melon.
Numbered embodiment 55. A di-haploid *Cucurbitaceae* plant, plant part, plant seed, or plant embryo produced by the method of any of Numbered embodiments 48-54, wherein the di-haploid plant is homozygous at substantially all genetic loci and is free of chimeric tissues.
Numbered embodiment 56. A method for accelerated selection of new commercial hybrid vegetable varieties, the method comprising:
   a) selecting elite F1 hybrid plants with broad genetic backgrounds and/or complementary agronomic traits;
   b) crossing the selected F1 hybrid plants to produce F2 populations;
   c) selecting top-performing F2 plants and performing whole genome sequencing to generate marker panel;
   d) producing di-haploid plants from the selected F2 plants using the methods of any of Numbered embodiments 37-47 or Numbered embodiments 48-54;
   e) subjecting the produced di-haploid plants to genotyping using heterozygous markers generated from the marker panel;
   f) selecting di-haploid lines with beneficial allele combinations using AI-assisted genomic selection;
   g) crossing the selected di-haploid lines to produce hybrid candidates; and
   h) validating heterosis performance in the hybrid candidates through field trials.
Numbered embodiment 57. The method of Numbered embodiment 56, wherein the selecting elite F1 hybrid plants in step a) comprises whole genome sequencing.
Numbered embodiment 58. The method of Numbered embodiment 56 or 57, wherein the agronomic trait is selected from the group consisting of yield, fruit quality, disease resistance, and stress tolerance, or any combination thereof.

### EXAMPLES

The below examples illustrate certain embodiments of the present disclosure. A person skilled in the art will appreciate that many modifications can be made in the specific examples which are disclosed and still obtain a similar result. Certain agents which are both chemically and physiologically related may be substituted for the agents described herein while achieving the same or similar results. All such substitutions and modifications apparent to those skilled in the art are deemed to be within the scope of the invention.

### Example 1: Production of Di-Haploid Pepper (Capsicum annuum) Plants through Androgenesis

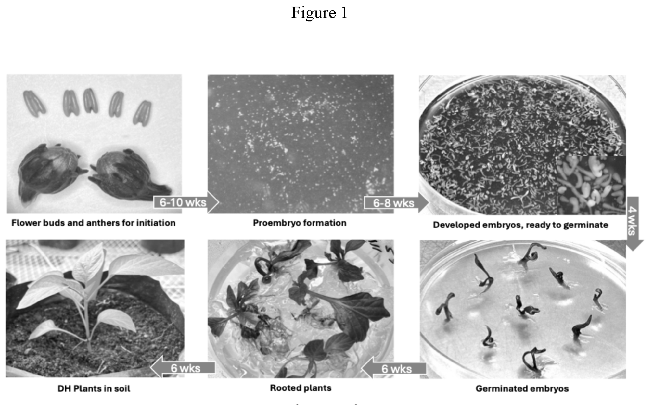

A procedure for producing DH/Di-H *Capsicum anuum* plants is outlined in Figure 1. The details in each step are described as follows.

### a) Flower bud selection, preparation and anther extraction

The donor plants were cultivated in the field or in a controlled environment until budding. A controlled environment such as a green house is desired due to less contamination with microorganisms. Flower buds, which were about 3-4 mm in length and bore anthers containing microspores, were collected, such as in the morning to maximize the quantity of flower buds containing microspores at a development stage amenable to embryogenesis. The microspores, when collected from the donor plants, are at a late uninucleate to early binucleate development stage. The plant material, in particular flower buds, can be collected at any time of the plant life cycle.

The collected flower buds were placed in a Ziploc bag with a pre-wet paper towel to maintain moisture. The buds were transported in an icebox to the lab, where they were first rinsed in 70% ethanol for 1 min. and immediately rinsed with sterile water three times. They were then surface-sterilized in a 1.0-1.4% sodium hypochlorite (20% Clorox Ultra) solution with agitation at a speed of 150 rpm for 10 minutes, followed by rinsing with sterile water three times. All the subsequent steps were performed under aseptic conditions.

Anthers were excised by gently opening the flower buds with forceps under a stereomicroscope and removing the filaments as much as possible without damaging the anthers.

### b) Anther inoculation and microspore release

Isolated anthers were cultured under conditions to facilitate releasing of healthy and large quantity of microspores. A double-layer culture medium system was used consisting of a semisolid under layer, and a liquid upper medium layer.

The semisolid under layer medium comprised of Nitsch and Nitsch (NN) medium (Cat. No. N616; PhytoTech Labs, Lenexa, KS 66215) supplemented with a range of 2%-5% maltose and 1% activated charcoal. After the pH of the medium was adjusted to 5.8, 0.6% plant agar (Cat. No. A111; PhytoTech Labs, Lenexa, KS 66215) was added. Following autoclaving at 121°C for 20 minutes, 4 mL aliquots of the medium were dispensed with a sterile serological pipette into 60 x 15 mm sterile Petri dishes and allowed to solidify.

All the anthers of a flower bud (generally 5-7 anthers) were inoculated randomly into a single Petri dish containing the semisolid under layer medium. After the anthers were inoculated, 4 mL of the liquid upper medium was gently dispensed over the under-layer medium in the Petri dish. The liquid upper layer medium (induction medium) comprised of Nitsch and Nitsch (NN) basal medium with vitamins supplemented with 2% maltose, a range of 0.05- 2 mg/L Zeatin, and 0.1-1 mg/L indole-3-acetic acid (IAA). At this stage, it was important that all the anthers were on the surface of the liquid medium. The petri dishes were sealed with a 2-3 cm stretch film stripe while the liquid medium was kept as still as possible. The sealed dishes were placed in a pre-sterilized plastic box.

### c) Culture of the released microspores for embryo formation

The anthers/microspores inoculated in the two-layer culture medium in Petri dishes from the previous step were cultured under conditions for embryo induction, development and plantlet regeneration.

Various parameters were evaluated to optimize microspore release, rate of embryo development, embryo competency for normal plant development, and spontaneous chromosome doubling. These include culture medium (such as basal salts, micronutrients, macronutrients, carbohydrates, vitamins and other additives such as absorbent materials), culture conditions (such as culture temperature and light condition), plant growth regulators such as auxins, cytokinins and their combinations. Different combinations of cytokinins and auxins were also tested alongside various pretreatments. Media P1-P4 in Table 2 shared the same base induction medium, i. e., Nitsch and Nitsch basal medium with vitamins (macro- and micronutrients, vitamins, amino acids, and 20 g/L maltose). The most effective treatment involved combining two auxins (2,4-D and IAA) and one cytokinin (Zeatin), along with a heat stress pretreatment. This combination consistently resulted in a significantly higher induction rate compared to other combinations (Table 2).

**Table 2. Effect of auxin/cytokinin combination and heat stress pretreatment treatment on double haploid plant production**

| Induction Medium | Auxin & Concentration (mg/L) | Cytokinin & Concentration (mg/L) | Stress Pretreatment | Stress Duration (days) | Average # of DH Plant per 100 Anthers |
|---|---|---|---|---|---|
| P1 | IAA (0.88) | Zeatin (0.55) | Heat (35°C) | 3-10 days | 19 |
| | | | Cold (8°C) | 5-10 days | 0 |
| P2 | 2,4-D (0.1) | Zeatin (0.55) | Heat (35°C) | 3-10 days | 31 |
| | IAA (0.88) | | Cold (8°C) | 5-10 days | 1 |
| P3 | 2,4-D (0.1) | Kinetin (0.2) | Heat (35°C) | 3-10 days | 1 |
| | | | Cold (8°C) | 5-10 days | 0 |
| P4 | 2,4-D (0.1) | Kinetin (0.2) | Heat (35°C) | 3-10 days | 2 |
| | | Zeatin (0.55) | Cold (8°C) | 5-10 days | 0 |

One of the parameters tested was exposing anthers to a stress pretreatment before culture or at the onset of the culture. The present inventors have surprisingly discovered that a stress pretreatment, such as a heat pretreatment applied to the anthers, before their culture or at the onset of the culture, increased the efficiency of embryogenesis from the released microspores in a genotype independent manner. For example, the anthers were exposed to a temperature of at least about 4 to about 12°C higher than the typical anther culture temperature of about 25°C for a duration of about 3 to about 10 days. In some embodiments, the anthers were exposed to a temperature of at least about 4 to about 12°C higher than the typical anther culture temperature of about 25°C for a duration of about 3 to about 10 days in the dark.

### d) Elongation of the embryos

Culture of the microspores under suitable conditions as described in the present disclosure generally resulted in the development of globular embryos. Globular embryos were then cultured under conditions effective to allow them undergo histodifferentiation, i.e., transition to torpedo and cotyledonary embryos. During this step, if more than 200 embryos emerged on the medium in a Petri dish, the embryos were then transferred into a new Petri dish with freshly prepared medium.

### e) Plantlet regeneration

The embryos with two well-formed cotyledons from the proceeding steps were selected and subcultured onto embryo germination medium until normal plantlet formation. The embryo germination medium contained Murashige and Skoog (MS) basal salts (Cat. No. M524, PhytoTech Labs, Lenexa, KS 66215; Murashige and Skoog, 1962), 3% sucrose, and 0.8% plant agar (Cat. No. A111; PhytoTech Labs, Lenexa, KS 66215). The culture was kept in a growth room at 25°C, with a photoperiod of 16-hour light (at the intensity of 50 µmol/m²/s)/8-hour dark until visible shoot elongation and differentiation of root and meristem regions were visible.

Seedlings showing cotyledons and roots were transferred onto a rooting medium in deli cups for further development. The rooting medium contained full strength MS basal salts, 0.05-0.5 mg/L Meta-Topolin and/or 0.01-0.1 mg/L gibberellic acid (GA₃), and 0.5%-1% plant agar. The plantlets were cultured at 25°C, with a photoperiod of 16-hour light (at the intensity of 50 µmol/m²/s)/8-hour dark.

### f) Plantlets in greenhouse

The plantlets with 3-7 leaves were transferred into pots filled with peat moss, perlite and vermiculite, and were placed into a mist chamber for 3-7 days with reduced lighting and high humidity to promote rooting. The plants were covered with wet paper towels and covered with a humidity dome. After two-three weeks, the plantlets were transferred into large pots for continuing growth to maturity and for sample collection for ploidy analyses.

### g) Analysis of the ploidy of the plants through flow cytometry

The plantlets that reached 5-10 leaf stage of development were analyzed for their ploidy level by flow cytometry, which allowed a reliable distinction between diploid (2n) and haploid (n) plants (Eeckhaut et al, 2005). Leaf samples were collected from expanded leaves of each plant and subjected to flow cytometry using Cyflow Space (Sysmex, Germany), a high-performance multi-laser flow cytometer, to determine the ploidy level of the plants. The ploidy level was analyzed according to the protocols provided by the manufacturer's manual. Several genotypes/market types of pepper were tested using the method of the present disclosure, and the results are summarized in Table 3. The "Market Type" column lists different genotypes tested; The "Sweet (S)/Pungent (P)" column indicates whether the pepper genotype have a mild flavor (sweet) a sharp, spicy flavor (pungent). The pungency of a pepper is determined by its capsaicinoid content, which can vary depending on the genotype and growing conditions. The "Total # Plants Produced" and "# Plants Tested" represent the total number of plants regenerated using the method in the present disclosure, and the number of plants subjected to flow cytometry analysis. "# Haploid Plants (n)" and "# Diploid Plants (2n)" represent the number of haploid plants and the numbers of spontaneously doubled haploid (SDH) plants based on the analysis by flow cytometry. "% SDH" shows the frequency of the spontaneously doubled haploid plants, which is defined as # Diploid Plants/# Plants Tested x 100.

As shown in Table 3, high frequencies of spontaneous chromosome doubling (% SDH), resulting in diploid plants, were obtained for all genotypes tested, ranging from 60% to 89%. The average spontaneous chromosome doubling frequency across all genotypes is 77%. The high spontaneous doubling frequency in combination with genotype independence of the present disclosure represents a major technology advancement in double haploid production for vegetable breeding to speed up variety development.

**Table 3. Analysis of ploidy level of regenerated plants from different pepper varieties**

| Market Type | Sweet(S)/ Pungent (P) | Total # Plants Produced | # Plants Tested | # Haploid Plants (n) | # Diploid Plants (2n) | % SDH |
|---|---|---|---|---|---|---|
| Bell Pepper | Sweet | 190 | 190 | 21 | 169 | 89 |
| Charleston | Sweet | 490 | 120 | 33 | 87 | 73 |
| Hot Banana | Highly Pungent | 95 | 87 | 13 | 74 | 85 |
| Kapya_Variety 1 | Sweet | 140 | 90 | 20 | 70 | 78 |
| Kapya_Variety 2 | Sweet | 250 | 140 | 56 | 84 | 60 |
| Anaheim | Mildly Pungent | 190 | 96 | 20 | 76 | 80 |
| Jalapeno_Variety 1 | Highly Pungent | 190 | 130 | 33 | 97 | 75 |
| Jalapeno_Variety 2 | Highly Pungent | 190 | 120 | 26 | 94 | 78 |

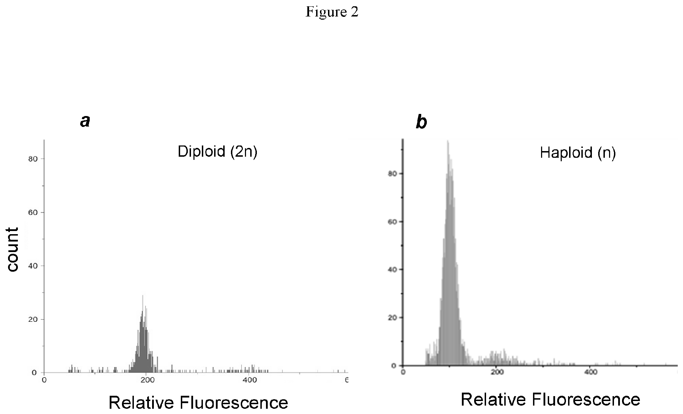

Figure 2 shows illustrative examples for ploidy analysis of the regenerated plants through flow cytometry. Figure 2(a) shows the flow cytometric histogram of DAPI (4', 6-diamidino-2-phenylindole, a blue-colored fluorescent dye that binds to DNA in the nuclei)-stained nuclei for a spontaneously doubled haploid (2n) pepper plant; and 2(b) shows the histogram for a haploid (n) pepper plant. As shown from the histograms, the di-haploid plant showed twice the fluorescence value as the haploid plant.

For varieties of Bell, Charleston, hot banana, Kapya_variety 1 and Kapya_variety 2, more than 150 seeds/per plant were harvested from all the diploid plants.

### h) Genotypic analysis of Double haploid Jalapeno plants from an F1 hybrid

This step served as both confirmation of the dihaploid nature of the regenerated plants and genotyping the population for designing crossing blocks guided by genomic selection model. The F1 plants were sequenced by Illumina^{®} sequencing and around 200 markers were selected across the genome from loci that were XY (i.e., heterozygous) in the hybrid.

If a regenerated diploid plant was produced from any maternal diploid plant cell of the female parent, it would continue to carry both alleles of the female parent at any given locus. Conversely, if the regenerated diploid plant was produced, as expected, through the individual microspore culture, it would carry only one allele at a given locus, presented in two identical copies. In one population derived from a given F1, each plantlet should exhibit either the XX or YY genotype at a given locus.

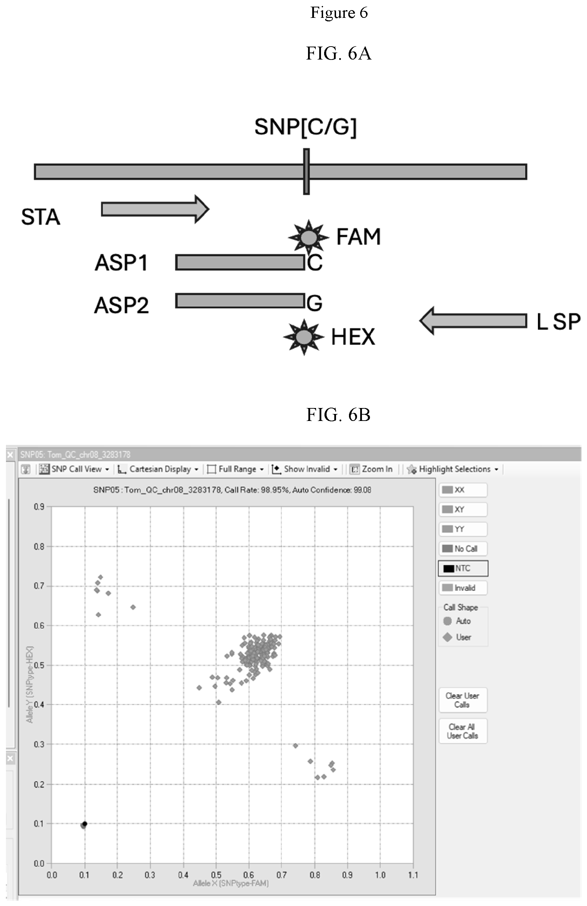

Leaf samples were taken from each plant and all the plants were numerically labelled for future pairing by genomic selection. DNA was extracted using the Wizard^{®} Magnetic 96 DNA Plant System by Promega (Madison, WI 53711). The genotyping was performed using Fluidigm^{®} SNP Type^{™} assay (Standard BioTools Inc., previously known as Fluidigm Corp., San Francisco, California 94080), which is based on allele-specific PCR SNP (single nucleotide polymorphism) detection chemistry, and provides a high throughput, low cost SNP genotyping solution that enables rapid design and polymorphism screening. The genotyping procedure consists of a pre-amplification step and a microfluid matrix chip assay. The Fluidigm^{®} SNP Type^{™} assay has three components: the Specific Target Amplification Primer (STA), which is used in a pre-amplification step to amplify a specific region of DNA containing the SNP of interest for further analysis, the Locus Specific Primer (LSP), which is used in conjunction with STA primer during the STA amplification step and help to specifically target the region containing the SNP, and the Allele Specific Primer (ASP), which is designed to bind to specific allele variations at the SNP site and is used in the main genotyping step, along with the STA amplified product, to differentiate between the different alleles (FIG. 6). These primers are purchased from Standard BioTools Inc. and used for amplifying and detecting specific regions of the DNA containing single nucleotide polymorphisms (SNPs). The use of the primers and probes are not critical and could be determined by a person skilled in the art using routine techniques. A pre-amplification primer pool consisted of both STA primers and LSP primers at a final concentration of 500 nM. The pre-amplification PCR mix consisted of 2.5 µL of Qiagen Master Mix, 0.5 µL of primer pool and 2 µL of sample DNA per sample reaction.

The pre-amplification PCR reaction consisted of several cycles of amplification as follows:
95°C for 15 minutes,
a 2 step 14 cycles of 95°C for 15 seconds,
60°C for 4 minutes.

The product from the pre-amplification was diluted to 200 µL final volume. 1.66 µL of the diluted product from pre-amplification was used for IFC chip assay following the manufacturer's protocol as follows:
1. Initial denaturation:
   - 95°C for 6 minutes,
2.Touchdown PCR (Gradient Annealing): 4 cycles
   - 95°C for 15 seconds
   - 64°C (decreasing by 1°C per cycle) for 45 seconds
   - 72°C for 15 seconds.
3. Amplification: 20 cycles
   ▪ 95°C for 15 seconds
   ▪ 60°C for 45 seconds
   ▪ 72°C for 15 seconds

   - Capture images after 20 cycles
4. Final cycling with Image Intervals: 5 cycles
   - 95°C for 15 seconds
   - 60 for 45 seconds
   - 72°C for 15 seconds

   - Capture images after 5 cycles and repeat Step 4 eight more times.

The image data were analyzed using Fluidigm^{®} SNP genotyping software.

Table 4 shows representative results of genotyping on 20 di-haploid plants (designated as P1, P2, etc.) derived from an F1. The results clearly show that the regenerated diploid plants were di-haploid plants. As expected, the F1 hybrid parent carried the XY alleles across all markers, whereas the regenerated di-haploid plants possessed either the XX or the YY alleles. Additionally, these plants exhibited distinct genotypes due to meiotic recombination. Chr stands for chromosome and L stands for locus. As an example, Chr 1_L3 refers to Locus 3 on Chromosome 1.

**Table 4. Confirmation on the di-haploid origin of the regenerated plants by genotyping**

| **Chromosome_Locus** | **Genotype** | | | | | | | | | | | | | | | | | | | | | |
|---|---|---|---|---|---|---|---|---|---|---|---|---|---|---|---|---|---|---|---|---|---|---|
| | **F1 Hybrid Parent** | | **P1** | **P2** | **P3** | **P4** | **P5** | **P6** | **P7** | **P8** | **P9** | **P10** | **P11** | **P12** | **P13** | **P14** | **P15** | **P16** | **P17** | **P18** | **P19** | **P20** |
| **Chr1_L1** | XY | YY | | YY | XX | YY | YY | YY | YY | YY | YY | YY | XX | XX | XX | YY | YY | XX | XX | YY | YY | XX |
| **Chr1_L2** | XY | YY | | YY | XX | YY | YY | YY | YY | YY | YY | YY | XX | XX | XX | YY | YY | XX | XX | YY | YY | XX |
| **Chr1_L3** | XY | YY | | YY | XX | YY | XX | YY | YY | XX | YY | YY | XX | XX | YY | YY | YY | XX | XX | XX | YY | XX |
| **Chr1_L4** | XY | XX | | XX | YY | XX | XX | XX | XX | XX | XX | XX | YY | YY | YY | XX | XX | YY | YY | XX | XX | YY |
| **Chr1_L5** | XY | YY | | YY | YY | YY | YY | YY | YY | YY | YY | YY | YY | XX | XX | XX | XX | XX | XX | XX | YY | XX |
| **Chr1_L6** | XY | YY | | YY | YY | YY | YY | YY | YY | YY | YY | YY | YY | XX | XX | XX | XX | XX | XX | XX | YY | XX |
| **Chr1_L7** | XY | XX | | YY | YY | YY | XX | YY | XX | XX | YY | XX | YY | XX | XX | XX | XX | YY | XX | XX | XX | XX |
| **Chr1_L8** | XY | YY | | YY | XX | XX | YY | YY | YY | YY | YY | YY | XX | YY | YY | YY | YY | YY | YY | YY | YY | YY |
| **Chr1_L9** | XY | YY | | YY | XX | YY | XX | YY | XX | XX | YY | YY | XX | XX | YY | YY | YY | XX | XX | XX | YY | XX |
| **Chr1_L10** | XY | XX | | XX | YY | XX | XX | XX | XX | XX | XX | XX | YY | YY | XX | XX | XX | YY | YY | XX | XX | YY |
| **Chr1_L11** | XY | YY | | YY | XX | YY | YY | YY | YY | YY | YY | YY | XX | XX | XX | YY | YY | XX | XX | YY | YY | XX |
| **Chr10_L12** | XY | YY | | YY | XX | YY | YY | YY | YY | YY | YY | YY | YY | YY | YY | YY | YY | YY | YY | YY | YY | YY |
| **Chr10_L13** | XY | XX | | XX | YY | XX | XX | XX | XX | XX | XX | XX | XX | XX | XX | XX | XX | XX | XX | XX | XX | XX |
| **Chr10_L14** | XY | YY | | YY | XX | YY | YY | YY | YY | YY | YY | YY | YY | YY | YY | YY | YY | YY | YY | YY | YY | YY |
| **Chr10_L15** | XY | YY | | YY | XX | YY | YY | YY | YY | YY | YY | YY | YY | YY | YY | YY | YY | YY | YY | YY | YY | YY |
| **Chr10_L16** | XY | YY | | YY | XX | YY | YY | YY | YY | YY | YY | YY | YY | YY | YY | YY | YY | YY | YY | YY | YY | YY |
| **Chr10_L17** | XY | XX | | YY | XX | YY | YY | YY | YY | XX | YY | YY | XX | YY | YY | YY | YY | YY | YY | YY | YY | YY |
| **Chr10_L18** | XY | XX | | XX | YY | XX | XX | XX | XX | XX | XX | XX | XX | XX | XX | XX | XX | XX | XX | XX | XX | XX |
| **Chr10_L19** | XY | XX | | XX | YY | XX | XX | XX | XX | XX | XX | XX | XX | XX | XX | XX | XX | XX | XX | XX | XX | XX |
| **Chr10_L20** | XY | XX | | XX | YY | XX | XX | XX | XX | XX | XX | XX | XX | XX | XX | XX | XX | XX | XX | XX | XX | XX |
| **Chr10_L21** | XY | XX | | XX | YY | XX | XX | XX | XX | XX | XX | XX | XX | XX | XX | XX | XX | XX | XX | XX | XX | XX |
| **Chr10_L22** | XY | XX | | XX | YY | XX | XX | XX | XX | XX | XX | XX | XX | XX | XX | XX | XX | XX | XX | XX | XX | XX |
| **Chr11_L23** | XY | YY | | XX | XX | YY | YY | XX | YY | XX | XX | YY | YY | XX | YY | YY | YY | XX | XX | XX | XX | XX |
| **Chr11_L24** | XY | YY | | XX | XX | YY | YY | XX | YY | XX | XX | YY | YY | XX | YY | YY | YY | XX | XX | XX | XX | XX |
| **Chr11_L25** | XY | YY | | XX | XX | YY | YY | XX | YY | XX | XX | XX | YY | XX | XX | YY | YY | XX | XX | YY | YY | XX |
| **Chr11_L26** | XY | YY | | XX | XX | YY | YY | XX | YY | XX | XX | YY | YY | XX | YY | YY | YY | XX | XX | XX | XX | XX |
| **Chr11_L27** | XY | YY | | XX | XX | YY | XX | XX | YY | XX | XX | YY | YY | XX | YY | YY | YY | XX | XX | XX | XX | XX |
| **Chr11_L28** | XY | XX | | YY | YY | XX | YY | YY | XX | YY | YY | XX | YY | YY | XX | YY | YY | YY | YY | YY | YY | YY |
| **Chr11_L29** | XY | YY | | XX | XX | YY | YY | XX | YY | XX | XX | XX | YY | XX | XX | YY | YY | XX | YY | YY | YY | XX |
| **Chr11_L30** | XY | YY | | XX | XX | YY | YY | XX | YY | XX | XX | YY | YY | XX | YY | YY | YY | XX | XX | XX | XX | XX |
| **Chr12_L31** | XY | YY | | YY | XX | XX | YY | YY | YY | XX | YY | XX | XX | XX | XX | XX | XX | XX | YY | XX | YY | YY |
| **Chr12_L32** | XY | YY | | YY | XX | XX | YY | YY | YY | XX | YY | XX | XX | XX | XX | XX | XX | XX | YY | XX | YY | YY |
| **Chr12_L33** | XY | YY | | YY | XX | XX | YY | YY | YY | XX | YY | XX | XX | XX | XX | XX | XX | YY | YY | XX | YY | YY |
| **Chr12_L34** | XY | YY | | YY | XX | XX | YY | YY | YY | XX | YY | XX | XX | XX | XX | XX | XX | YY | YY | XX | YY | YY |
| **Chr12_L35** | XY | YY | | YY | XX | XX | YY | YY | YY | XX | YY | XX | YY | XX | XX | XX | XX | XX | YY | XX | XX | XX |
| **Chr12_L36** | XY | YY | | YY | XX | XX | YY | YY | YY | XX | YY | XX | XX | XX | XX | XX | XX | YY | YY | XX | YY | YY |
| **Chr12_L37** | XY | YY | | YY | XX | XX | YY | YY | YY | XX | YY | XX | XX | XX | XX | XX | XX | YY | YY | XX | YY | YY |
| **Chr12_L38** | XY | XX | | YY | XX | XX | YY | YY | YY | YY | YY | XX | YY | XX | XX | XX | XX | YY | YY | XX | YY | YY |
| **Chr12_L39** | XY | XX | | XX | YY | YY | XX | XX | XX | YY | XX | YY | YY | YY | YY | YY | YY | YY | XX | YY | XX | YY |
| **Chr12_L40** | XY | YY | | YY | XX | XX | YY | YY | YY | XX | YY | XX | XX | XX | XX | XX | XX | XX | YY | XX | YY | YY |
| **Chr12_L41** | XY | YY | | YY | XX | XX | YY | YY | YY | XX | YY | XX | XX | XX | XX | XX | XX | XX | YY | XX | YY | YY |
| **Chr12_L42** | XY | YY | | XX | YY | XX | YY | XX | XX | XX | XX | XX | YY | YY | XX | XX | XX | XX | YY | XX | XX | XX |
| **Chr12_L43** | XY | YY | | YY | XX | XX | YY | YY | YY | XX | YY | XX | XX | XX | XX | XX | XX | XX | YY | XX | YY | YY |
| **Chr2_L44** | XY | YY | | XX | YY | YY | YY | XX | YY | XX | XX | YY | YY | XX | YY | YY | YY | XX | YY | XX | YY | YY |
| **Chr2_L45** | XY | YY | | YY | YY | YY | YY | YY | YY | YY | YY | YY | YY | XX | YY | YY | YY | XX | YY | XX | YY | YY |
| **Chr2_L46** | XY | XX | | XX | XX | XX | XX | XX | XX | XX | XX | XX | XX | YY | XX | XX | XX | YY | XX | YY | XX | XX |
| **Chr2_L47** | XY | YY | | YY | YY | YY | YY | YY | YY | XX | YY | YY | YY | YY | YY | XX | XX | YY | YY | XX | YY | YY |
| **Chr2_L48** | XY | XX | | XX | XX | XX | YY | XX | XX | YY | XX | XX | YY | XX | XX | YY | YY | YY | XX | YY | XX | XX |
| **Chr2_L49** | XY | XX | | YY | XX | XX | YY | YY | XX | YY | YY | YY | XX | YY | YY | XX | XX | YY | XX | YY | XX | XX |
| **Chr2_L50** | XY | XX | | YY | XX | XX | YY | YY | XX | YY | YY | YY | XX | YY | YY | XX | XX | YY | XX | YY | XX | XX |
| **Chr2_L51** | XY | YY | | XX | YY | YY | XX | XX | YY | XX | XX | XX | YY | XX | XX | YY | YY | XX | YY | XX | YY | YY |
| **Chr2_L52** | XY | YY | | XX | YY | YY | XX | XX | YY | XX | XX | XX | YY | XX | XX | YY | YY | XX | YY | XX | YY | YY |
| **Chr2_L53** | XY | YY | | XX | YY | YY | XX | XX | YY | XX | XX | XX | YY | XX | XX | YY | YY | XX | YY | XX | YY | YY |
| **Chr2_L54** | XY | YY | | XX | YY | YY | YY | XX | YY | XX | XX | YY | YY | XX | YY | YY | YY | XX | YY | XX | YY | YY |
| **Chr3_L55** | XY | XX | | YY | XX | XX | XX | YY | XX | XX | YY | XX | YY | XX | XX | XX | XX | XX | YY | YY | XX | XX |
| **Chr3_L56** | XY | XX | | YY | XX | XX | XX | YY | XX | XX | YY | XX | YY | XX | XX | XX | XX | XX | YY | YY | XX | XX |
| **Chr3_L57** | XY | XX | | YY | XX | XX | XX | YY | XX | XX | YY | XX | YY | XX | XX | XX | XX | XX | YY | YY | XX | XX |
| **Chr3_L58** | XY | XX | | YY | XX | XX | XX | YY | XX | XX | YY | XX | YY | XX | XX | XX | XX | XX | YY | YY | XX | XX |
| **Chr3_L59** | XY | XX | | YY | XX | XX | XX | YY | XX | XX | YY | XX | YY | XX | XX | XX | XX | XX | YY | YY | XX | XX |
| **Chr3_L60** | XY | XX | | YY | XX | XX | XX | YY | XX | XX | YY | XX | YY | XX | XX | XX | XX | XX | YY | YY | XX | XX |
| **Chr3_L61** | XY | XX | | YY | XX | XX | XX | YY | XX | XX | YY | XX | YY | XX | XX | XX | XX | XX | YY | YY | XX | XX |

### Example 2: AI-Guided Pepper F1 Crosses Showed Enhanced Yield Compared to the Original F1 Hybrid

Two commercial jalapeno hybrids were used in this example. *Hybrid*_*1* carries a disease resistance gene but produces smaller-size fruits, while *Hybrid*_*2* yields well-sized fruits but lacks the resistance allele. A common breeding strategy is to self *Hybrid*_*1* and use marker-assisted selection to fix the resistance gene. After 5-7 generations of selfing, breeders typically design hundreds of crossing blocks based on their experience and phenotypic performance of inbred lines, followed by extensive field testing. This process is time consuming and cost prohibitive for smaller corporations. Moreover, it may overlook valuable heterotic combinations due to undesirable traits present in one of the parents.

In this example, the two commercial hybrids were crossed to introduce the resistance allele into the F2 population. From approximately 700 recombinants, 12 top-performing lines were selected based on fruit size, fruit settings, pungency, and molecular markers for the targeted resistance allele. These 12 lines were micropropagated into five clones each and subsequently narrowed down into three elite selections. These three lines will generate 100-200 doubled haploid plants to reversely reconstitute the desirable F2 hybrid.

Table 5 provides greenhouse data on six F2 (Plant ID 6-25) lines compared to their parents (F1, parent 1, Plant ID 1-5 and F1, parent 2, plant ID 36-40). The progeny displayed larger fruit size, higher yield, earlier flowering, and reduced flower abortion relative to both parents. These results, as shown in Table 5, indicate that this described method can significantly enhance the probability of identifying the next commercial hybrid by using meiotic directed recombination.

**Table 5. AI-guided pepper F1 crosses in combination with double haploid production led to enhanced yield compared to the original F1 hybrids**

| Plant ID | Generation | Average Fruit length (inch) | Average Fruit Weight (g) | First Fruit Set (days after transplant) | Fruit Number (60 days after transplanting) |
|---|---|---|---|---|---|
| 1 | F1 (Parent 1) | 4.1 | 45.03 | 18 Days | 18 |
| 2 | F1 (Parent 1) | 4.3 | 47.58 | 18 Days | 18 |
| 3 | F1 (Parent 1) | 4.2 | 46.32 | 19 Days | 19 |
| 4 | F1 (Parent 1) | 4.1 | 44.96 | 17 Days | 18 |
| 5 | F1 (Parent 1) | 4.1 | 44.84 | 17 Days | 18 |
| 6 | F2 (25JAL_01) | 4.6 | 52.40 | 15 Days | 20 |
| 7 | F2 (25JAL_01) | 4.5 | 51.73 | 15 Days | 19 |
| 8 | F2 (25JAL_01) | 4.7 | 54.02 | 15 Days | 21 |
| 9 | F2 (25JAL_01) | 4.5 | 51.67 | 15 Days | 21 |
| 10 | F2 (25JAL_01) | 4.7 | 53.89 | 15 Days | 21 |
| 11 | F2 (25JAL_02) | 5.2 | 66.31 | 14 Days | 21 |
| 12 | F2 (25JAL_02) | 5.0 | 63.77 | 15 Days | 22 |
| 13 | F2 (25JAL_02) | 5.1 | 64.25 | 14 Days | 21 |
| 14 | F2 (25JAL_02) | 5.1 | 64.80 | 15 Days | 20 |
| 15 | F2 (25JAL_02) | 5.2 | 66.11 | 15 Days | 21 |
| 16 | F2 (25JAL_03) | 4.8 | 56.32 | 15 Days | 20 |
| 17 | F2 (25JAL_03) | 4.8 | 56.18 | 15 Days | 20 |
| 18 | F2 (25JAL_03) | 4.9 | 59.01 | 16 Days | 19 |
| 19 | F2 (25JAL_03) | 4.9 | 58.34 | 15 Days | 21 |
| 20 | F2 (25JAL_03) | 4.8 | 56.68 | 14 Days | 20 |
| 21 | F2 (25JAL_04) | 4.7 | 55.27 | 15 Days | 21 |
| 22 | F2 (25JAL_04) | 4.6 | 54.01 | 15 Days | 22 |
| 23 | F2 (25JAL_04) | 4.7 | 54.97 | 15 Days | 20 |
| 24 | F2 (25JAL_04) | 4.6 | 54.3 | 15 Days | 19 |
| 25 | F2 (25JAL_04) | 4.6 | 54.4 | 15 Days | 20 |
| 26 | F2 (25JAL_05) | 5.2 | 65.32 | 14 Days | 20 |
| 27 | F2 (25JAL_05) | 5.3 | 66.41 | 15 Days | 20 |
| 28 | F2 (25JAL_05) | 5.2 | 64.20 | 15 Days | 19 |
| 29 | F2 (25JAL_05) | 5.3 | 65.74 | 14 Days | 20 |
| 30 | F2 (25JAL_05) | 5.2 | 65.1 | 14 Days | 21 |
| 31 | F2 (25JAL_06) | 5.2 | 69.87 | 15 Days | 20 |
| 32 | F2 (25JAL_06) | 5.3 | 71.07 | 15 Days | 19 |
| 33 | F2 (25JAL_06) | 5.3 | 70.43 | 16 Days | 19 |
| 34 | F2 (25JAL_06) | 5.1 | 68.03 | 15 Days | 20 |
| 35 | F2 (25JAL_06) | 5.3 | 71.4 | 15 Days | 20 |
| 36 | F1 (Parent 2) | 3.8 | 45.03 | 15 Days | 21 |
| 37 | F1 (Parent 2) | 3.7 | 44.77 | 15 Days | 20 |
| 38 | F1 (Parent 2) | 3.9 | 47.61 | 15 Days | 22 |
| 39 | F1 (Parent 2) | 3.8 | 46.34 | 15 Days | 19 |
| 40 | F1 (Parent 2) | 3.9 | 49.61 | 15 Days | 20 |

### Example 3: Production of Di-Haploid Tomato (Lycopersicon esculentum) Plants through Androgenesis

### a) Flower bud selection, preparation and anther extraction

A similar method as described for *Capsicum annuum* in Example 1 was used for flower bud selection. Briefly, donor plants were cultivated either in the field or in a controlled environment until the budding stage. A controlled environment, such as a greenhouse, was used to reduce the risk of microbial contamination. Flower buds measuring approximately 3-4 mm in length, with anthers containing microspores, were collected, such as in the morning - to maximize the yield of microspores at a development stage amenable to embryogenesis. In some embodiments, microspores are collected at the late uninucleate to early binucleate stage. The plant material, particularly flower buds, can be collected at any time of the plant life cycle.

The collected flower buds were placed in a Ziploc bag with a pre-wet paper towel to maintain moisture. The buds were transported to the lab in an icebox. Upon arrival, the buds were rinsed in 70% ethanol for 1 min, followed by three washes with sterile water. Next, they were surface sterilized in a 1.0-1.4% sodium hypochlorite (20% Clorox Ultra) solution with agitation at 150 rpm for 10 minutes, then rinsed three times with sterile water. All the subsequent steps were performed under aseptic conditions.

### b) Anther/microspore culture for embryogenesis

The flower buds were carefully dissected to ensure the complete removal of filaments using a scalpel and tweezers. Anthers with a purple hue contain the most uninucleate microspores, which are the most responsive for embryo induction.

Two to ten anthers were transferred to a Petri dish containing embryo induction medium containing Murashige & Skoog Basal Medium with Vitamins (Cat. No. M519, PhytoTech Labs, Lenexa, KS 66215) supplemented with 0.02-5 µM 2,4-dichlorophenoxyacetic acid (2,4 D), 0.1 mg/L Vitamin B12, 30 g/L sucrose, and 8 g/L of agar (Cat. No. H26724-22, VWR). Each Petri dish was filled with 5-10 mL of embryo induction medium. The anthers were then incubated in the dark at 4°C for 2 days, followed by incubation in a temperature-controlled environment and maintained at 35°C in the dark for 1-10 days.

After heat stress pretreatment at 35°C, the Petri dishes containing the tomato anthers were moved to a growth chamber and incubated under a 16-hour light (50 µmolm⁻²s⁻¹)/8-hour dark photoperiod at 25°C for four to six weeks when calli began to emerge from both ends of the anther. After 6-8 weeks, embryos started to appear on the calli at both ends of the anthers.

### c) Plantlet development

The emerged embryos with the associated calli were gently transferred with fine tweezers into Petri dishes containing germination medium (Gamborg B-5 basal medium, 30 g/L sucrose, 0.01-0.3 mg/L zeatin, and 8 g/L agar), and incubated in a growth chamber at 25°C under a 16-hour light (50 µmolm⁻²s⁻¹)/8-hour dark photoperiod for 3-10 weeks.

*The following steps describe a prophetic procedure based on the teachings of the present disclosure.*

The individual plantlets developed on the germination medium are transferred into a rooting medium containing half strength of Murashige & Skoog Basal Medium, 30 g/L sucrose, 0.4-4 µM IAA, and 6 g/L gelrite.

The plantlets with 3-7 leaves are transferred into pots filled with peat moss, perlite and vermiculite, and are placed into a mist chamber for 3-7 days with reduced lighting and high humidity to promote rooting. The plants are covered with wet paper towels and covered with a humidity dome. After two-three weeks, the plantlets are transferred into large pots for continuing growth to maturity and for sample collection for ploidy analyses.

### d) Analysis of the ploidy of the plants through flow cytometry

Plantlets that reached 5-10 leaf stage of development are analyzed for their ploidy level by flow cytometry. Leaf samples are collected from expanded leaves of each plant and subjected to flow cytometry using Cyflow Space (Sysmex, Germany), a high-performance multi-laser flow cytometer, to determine the ploidy level of the plants. The ploidy level is analyzed according to the protocols provided by the manufacturer's manual.

### Example 4: Production of Di-Haploid Solanum melongena Plants through Androgenesis

### a) Flower bud selection, preparation and anther extraction

A similar method as described for *Capsicum annuum* in Example 1 was used for flower bud selection. Briefly, donor plants were cultivated either in the field or in a controlled environment until the budding stage. A controlled environment, such as a greenhouse, was used to reduce the risk of microbial contamination. Flower buds totally closed with petals fully covered by sepals were selected for anther culture. The buds were excised and kept in plastic tubes in an ice box.

Upon arrival in the laboratory, the buds were rinsed in 70% ethanol for 1 min, followed by three washes with sterile water. Next, they were surface sterilized in a 1.0-1.4% sodium hypochlorite (20% Clorox Ultra) solution with agitation at 150 rpm for 10 minutes, then rinsed three times with sterile water. All the subsequent steps were performed under aseptic conditions.

### b) Anther Culture for Embryogenesis

The anther filaments were completely removed, and twenty anthers were placed on induction medium comprising Eggplant Basal Medium (Table 1-1, Calabuig-Serna et al., 2021) supplemented with 5 mg/L Kinetin, 5 mg/L 2,4-D, 0.2 mg/L B12 vitamin, 120 g/L sucrose, and 8 g/L agar. The anthers were incubated in the dark at 35°C for 5-10 days, depending on the genotype. Subsequently, they were transferred to a 25°C growth room under a 16-hour light/ 8-hour dark photoperiod for additional 4 days.

The anthers were transferred to the germination medium (Table 6) and maintained under the same conditions until embryos emerged from the necrotic anther tissue. Once the embryos develop visible cotyledons, they are transferred to rooting medium (Table 6) and maintained until the seedlings form 4-5 true leaves and well-developed roots. The seedlings are then transplanted into soil-filled pots and acclimatized using a plastic cup and a damped paper towel for 2-5 days. After two-three weeks, the plantlets are transferred into large pots for continuing growth to maturity and for sample collection for ploidy analyses, similar to as described in Example 1g.

**Table 6. Eggplant medium composition for di-haploid plant production**

| Ingredient | Induction Basal Medium | Germination Medium | Rooting Medium |
|---|---|---|---|
| *Macroelements* | Final Concentration (mg/L) | | |
| KNO3 | 2150 | 2150 | 1900 |
| NH4 NO3 | 1238 | 1238 | 1650 |
| MgSO4·7H2O | 412 | 412 | 370 |
| CaCl2·2H2O | 313 | 313 | 440 |
| KH2PO4 | 142 | 142 | 170 |
| Ca(NO3)2·4H2O | 50 | 50 | - |
| NaH2PO4·H2O | 38 | 38 | - |
| (NH4)2SO4 | 34 | 34 | - |
| KCl | 7 | 7 | - |

| *Microelements* | | | |
|---|---|---|---|
| MnSO4·H2O | 22.13 | 20.13 | 0.076 |
| ZnSO₄·7H₂O | 3.625 | 3.225 | 1 |
| H₃BO₃ | 3.15 | 1.55 | 1 |
| KI | 0.695 | 0.33 | 0.01 |
| Na₂MoO₄·2H₂O | 0.188 | 0.138 | - |
| CuSO₄·5H₂O | 0.016 | 0.011 | 0.03 |
| CoCl₂·6H₂O | 0.016 | 0.011 | - |
| AlCl₃·6H₂O | - | - | 0.05 |
| NiCl₂·6H₂O | - | - | 0.03 |

| *Vitamins and amino acids* | | | |
|---|---|---|---|
| Myo-inositol | 100 | 100 | - |
| Pyridoxine HCl | 5.5 | 5.5 | - |
| Nicotinic acid | 0.7 | 0.7 | - |
| Thiamine HCl | 0.6 | 0.6 | - |
| Calcium pantothenate | 0.5 | 0.5 | - |
| Biotin | 0.005 | 0.005 | - |
| Sucrose | 120 g/L | 30 g/L | 30 g/L |

### Example 5: High-Efficiency Spontaneous Chromosome Doubling Across Diverse Eggplant (Solanum melongena) Market Types

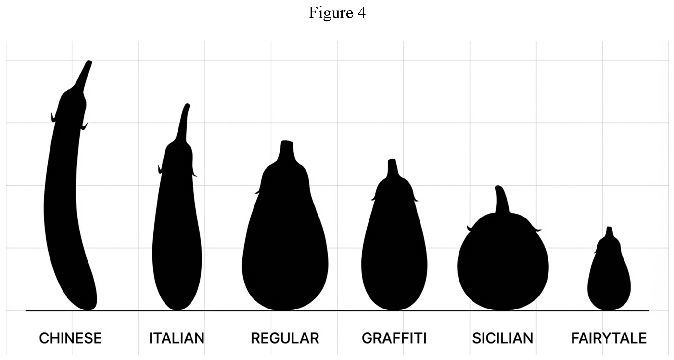

To verify the efficiency and genotype-independent nature of the disclosed androgenesis method for eggplant, multiple distinct eggplant market types and germplasms (based on eggplant fruit shapes as shown in Figure 4) were subjected to the di-haploid production protocol described in Example 4. Plantlets reaching the 5- to 10-leaf stage were analyzed for their ploidy level using flow cytometry to distinguish between haploid (n) and spontaneously doubled diploid (2n) plants.

The results of the analyses are summarized in Table 7. Surprisingly, high frequencies of spontaneous chromosome doubling (SDH) across all evaluated eggplant varieties, ranging from 79% to 89%, were observed. % SDH shows the frequency of the spontaneously doubled haploid plants, defined as (# diploid plants/# plants tested) x 100. This high spontaneous doubling efficiency eliminates the need for toxic chemical chromosome-doubling agents (such as colchicine), significantly streamlining the breeding process and establishing a highly efficient di-haploid production pipeline for eggplant.

**Table 7. Analysis of Ploidy Level of Regenerated Plants from Different Eggplant Varieties**

| Market type | Total # Plants Produced | #Plants Tested | # Haploid Plants (n) | # Diploid Plants (2n) | % SDH |
|---|---|---|---|---|---|
| Chinese eggplant | 30 | 30 | 5 | 25 | 83 |
| Sicilian eggplant | 50 | 50 | 10 | 40 | 80 |
| Italian type Eggplant | 38 | 38 | 6 | 32 | 84 |
| Graffiti Eggplant_germplasm 1 | 57 | 57 | 11 | 46 | 80 |
| Graffiti Eggplant_germplasm 2 | 24 | 24 | 5 | 19 | 79 |
| Graffiti Eggplant_germplasm 3 | 96 | 96 | 14 | 82 | 85 |
| Graffiti Eggplant_germplasm 4 | 47 | 47 | 5 | 42 | 89 |

To further confirm the di-haploid origin of the regenerated eggplant lines and to verify complete homozygosity, genotypic analysis was conducted on 21 doubled haploid plants (designated as eggplant_graffiti_DH_001 through 021) derived from Graffiti eggplant germplasm. Genomic DNA was extracted and analyzed using a customized panel of 18 SNP markers (Eggplant_DH_marker_01 through 18), following the same protocol as described in Example 1.

True di-haploid plants, originating from individual microspores that undergo spontaneous chromosome doubling, possess only a single parental allele at each locus, present as two identical copies. Accordingly, such plants are expected to display exclusively homozygous genotypes (either XX or YY) at loci that are heterozygous in the donor parent, with no heterozygous (XY) call detected.

As shown in Table 8, all 21 evaluated Graffiti eggplant plants exhibited strictly homozygous genotypes (either XX or YY) across all 18 markers. Moreover, the observed segregation of XX and YY alleles among the plants is consistent with meiotic recombination. Collectively, these results confirm that the method reliably generates true, fully homozygous di-haploid eggplant lines suitable for direct use in commercial hybrid breeding programs.

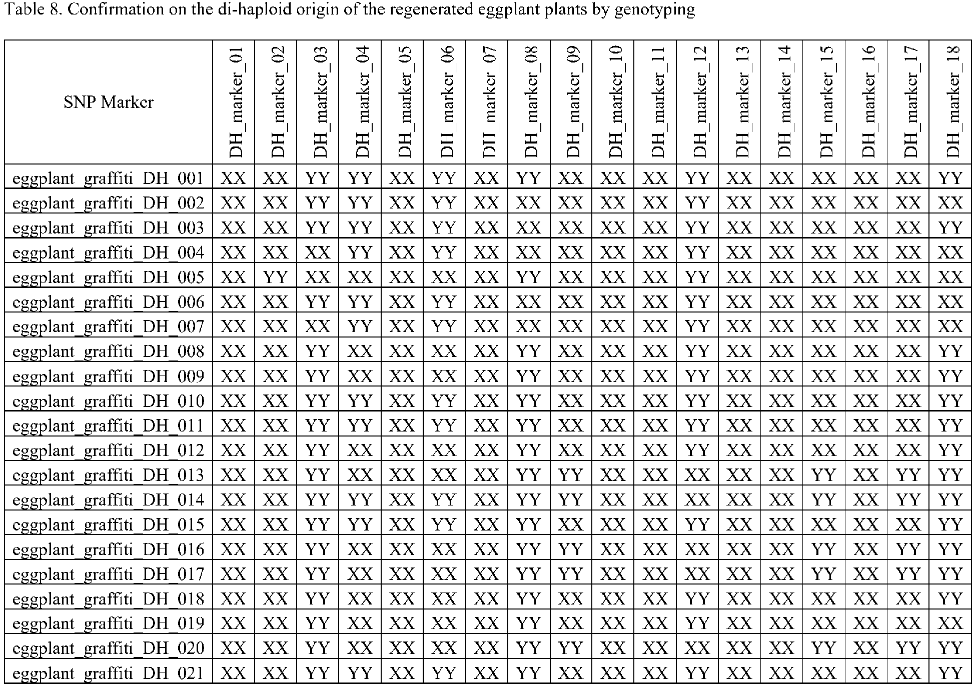

### Example 6: Production of Di-Haploid Cucumber (Cucumis sativus) Plants through Gynogenesis

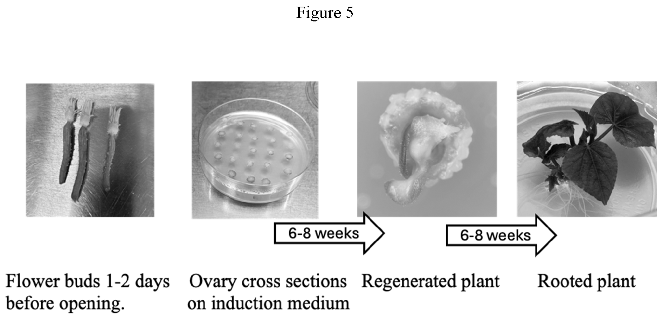

The process for efficient production of di-haploid *Cucumis sativus* through gynogenesis is outlined in Figure 5. The details in each step are described as follows.

### a) Flower bud selection, preparation and ovary collection

The donor plants were cultivated in the field or in a controlled environment until budding. A controlled environment, such as a green house, is used due to the reduced risk of microbial contamination. Ovaries were selected from donor plants one month after the emergence of the second female flower. Unfertilized ovaries were collected six hours before anthesis. The corolla exhibited a yellow-green hue, and most ovaries contained nearly mature embryonic sacs, making them ideal explants for inducing in vitro gynogenesis. The harvested ovaries were used either fresh or after being pretreated at 4°C in a refrigerator.

Following the removal of the papilla, 6-10 ovaries were surface sterilized by immersing them in 70% ethanol for 60 seconds, followed by soaking them in 1% sodium hypochlorite for 20 minutes. The ovaries were then rinsed three times with sterile distilled water.

### c) Ovary culture and plantlet regeneration

After surface sterilization, the ovaries were sliced into 2 mm cross-sections under sterile conditions and placed in 150 mL Petri dishes containing 30-70 mL of induction medium comprising Murashige & Skoog Basal Medium with vitamins (Cat. No. M519, PhytoTech Labs, Lenexa, KS 66215), supplemented with 0.01-0.5 mg/L Thidiazuron (TDZ), and 30 g/L sucrose.

The cultures were incubated in the dark at 28-35°C for 1-10 days to undergo heat stress pretreatment. Following the treatment, they were transferred to a growth chamber under controlled conditions: a 16-hour light (50 µmolm⁻²s⁻¹)/8-hour dark photoperiod at 25°C, where they remained under the same condition until plantlets emerged.

Seedlings showing the first true leaf and roots were transferred into rooting medium in deli cups for further development. The rooting medium contained full strength MS basal salts, 0.05-0.5 mg/L Meta-Topolin [6-(3-hydroxybenzylamino)purine, an aromatic cytokinin], and/or 0.01-0.1 mg/L gibberellic acid (GA₃), 30 g/L sucrose, and 0.5%-1% plant agar. The plantlets were cultured at 25°C, with a photoperiod of 16-hour light (at the intensity of 50 µmol/m²/s)/8-hour dark.

### d) Plants in greenhouse

Plantlets with 3-7 leaves were transferred into pots filled with a mixture of peat moss, perlite, and vermiculite, and placed in a mist chamber for 3-7 days under reduced lighting and high humidity to promote rooting. The plantlets were covered with wet paper towels and enclosed within a humidity dome. After seven days, the paper towels and humidity dome were removed, allowing the plantlets to grow under normal greenhouse conditions. After two to three weeks, the plants were transferred into larger pots for continued growth to maturity and for sample collection for ploidy analysis.

### e) Ploidy test and genotypic analysis of the regenerated plants

Similar methods were used to determine the ploidy level of the regenerated cucumber plants as described in Example 1. Briefly, the ploidy level of plantlets at the 5-10 leaf stage was assessed using flow cytometry to differentiate between diploid (2n) and haploid (n) plants, following the method described by Eeckhaut et al. (2005). Leaf samples were collected from fully expanded leaves and processed for flow cytometry analysis. The ploidy determination was conducted according to the manufacturer's protocol (Cyflow Space, Sysmex, Germany).

As shown in Table 9, high frequencies of spontaneous double haploid were achieved in multiple hybrids from two varieties, similar to the results in pepper as described in Example 1.

**Table 9. Frequency of spontaneous double haploid in Cucumis sativus Plants through Gynogenesis**

| **Market Type** | **Total # Plants Produced** | **# Plants Tested** | # **Haploid Plants** (n) | **# Diploid Plants (2n)** | **% SDH** |
|---|---|---|---|---|---|
| Slicer Cucumber_Variety 1 | 15 | 15 | 3 | 12 | 80 |
| Slicer Cucumber_Variety 2 | 20 | 20 | 2 | 18 | 90 |
| Dutch Cucumber_Variety 1 | 10 | 10 | 0 | 10 | 100 |
| Dutch Cucumber_Variety 2 | 6 | 6 | 1 | 5 | 80 |

Genotyping of cucumber double haploid plants was performed similar to that described in Example 1. Leaf samples were taken from each plant, and all the plants were numerically labelled for future pairing by genomic selection. DNA was extracted using the Wizard^{®} Magnetic 96 DNA Plant System by Promega (Madison, WI 53711). Genotyping was carried out using Fluidigm^{®} SNP Type^{™} assay, a high throughput, low-cost SNP genotyping method based on allele-specific PCR SNP (single nucleotide polymorphism) detection chemistry. This approach enables rapid assay design and efficient polymorphism screening. Sequencing data confirmed a lower level of polymorphisms within the genome of cucumber hybrids and cucumber has a smaller size of genome (data not shown). Based on these findings, a panel of twenty-four SNP markers was selected for genotyping cucumber double haploid plants. The results demonstrated that the double haploid plants (designated as P1, P2, P3, etc) carried homozygous alleles at all 24 SNP loci and exhibited either XX or YY genotypes for loci that were heterozygous in the F1 generation (Table 10).

**Table 10. Confirmation on the di-haploid origin of the regenerated Cucumis sativus plants by genotyping**

| | | | | | | | | | | | | | | | | | | | | | | |
|---|---|---|---|---|---|---|---|---|---|---|---|---|---|---|---|---|---|---|---|---|---|---|
| **Genomic Locus** | **Genotype** | | | | | | | | | | | | | | | | | | | | | |
| | **Hybrid F1** | **P1** | **P2** | **P3** | **P4** | **P5** | **P6** | **P7** | **P8** | **P9** | **P10** | **P11** | **P12** | **P13** | **P14** | **P15** | **P16** | **P17** | **P18** | **P19** | **P20** | **P21** |
| **Locus 1** | XX | XX | XX | XX | XX | XX | XX | XX | XX | XX | XX | XX | XX | XX | XX | XX | XX | XX | XX | XX | XX | XX |
| **Locus 2** | XX | XX | XX | XX | XX | XX | XX | XX | XX | XX | XX | XX | XX | XX | XX | XX | XX | XX | XX | XX | XX | XX |
| **Locus 3** | XY | YY | XX | YY | YY | YY | XX | YY | YY | XX | YY | YY | YY | YY | YY | YY | YY | YY | YY | YY | YY | YY |
| **Locus 4** | XX | XX | XX | XX | XX | XX | XX | XX | XX | XX | XX | XX | XX | XX | XX | XX | XX | XX | XX | XX | XX | XX |
| **Locus 5** | YY | YY | YY | YY | YY | YY | YY | YY | YY | YY | YY | YY | YY | YY | YY | YY | YY | YY | YY | YY | YY | YY |
| **Locus 6** | XX | XX | XX | XX | XX | XX | XX | XX | XX | XX | XX | XX | XX | XX | XX | XX | XX | XX | XX | XX | XX | XX |
| **Locus 7** | XX | XX | XX | XX | XX | XX | XX | XX | XX | XX | XX | XX | XX | XX | XX | XX | XX | XX | XX | XX | XX | XX |
| **Locus 8** | YY | YY | YY | YY | YY | YY | YY | YY | YY | YY | YY | YY | YY | YY | YY | YY | YY | YY | YY | YY | YY | YY |
| **Locus 9** | XX | XX | XX | XX | XX | XX | XX | XX | XX | XX | XX | XX | XX | XX | XX | XX | XX | XX | XX | XX | XX | XX |
| **Locus 10** | YY | YY | YY | YY | YY | YY | YY | YY | YY | YY | YY | YY | YY | YY | YY | YY | YY | YY | YY | YY | YY | YY |
| **Locus 11** | YY | YY | YY | YY | YY | YY | YY | YY | YY | YY | YY | YY | YY | YY | YY | YY | YY | YY | YY | YY | YY | YY |
| **Locus 12** | YY | YY | YY | YY | YY | YY | YY | YY | YY | YY | YY | YY | YY | YY | YY | YY | YY | YY | YY | YY | YY | YY |
| **Locus 13** | YY | YY | YY | YY | YY | YY | YY | YY | YY | YY | YY | YY | YY | YY | YY | YY | YY | YY | YY | YY | YY | YY |
| **Locus 14** | XX | XX | XX | XX | XX | XX | XX | XX | XX | XX | XX | XX | XX | XX | XX | XX | XX | XX | XX | XX | XX | XX |
| **Locus 15** | XY | XX | XX | XX | XX | XX | XX | XX | XX | XX | XX | XX | XX | XX | XX | XX | XX | XX | YY | XX | XX | XX |
| **Locus 16** | YY | YY | YY | YY | YY | YY | YY | YY | YY | YY | YY | YY | YY | YY | YY | YY | YY | YY | YY | YY | YY | YY |
| **Locus 17** | YY | YY | YY | YY | YY | YY | YY | YY | YY | YY | YY | YY | YY | YY | YY | YY | YY | YY | YY | YY | YY | YY |
| **Locus 18** | XX | XX | XX | XX | XX | XX | XX | XX | XX | XX | XX | XX | XX | XX | XX | XX | XX | XX | XX | XX | XX | XX |
| **Locus 19** | YY | YY | YY | YY | YY | YY | YY | YY | YY | YY | YY | YY | YY | YY | YY | YY | YY | YY | YY | YY | YY | YY |
| **Locus 20** | XX | XX | XX | XX | XX | XX | XX | XX | XX | XX | XX | XX | XX | XX | XX | XX | XX | XX | XX | XX | XX | XX |
| **Locus 21** | XX | XX | XX | XX | XX | XX | XX | XX | XX | XX | XX | XX | XX | XX | XX | XX | XX | XX | XX | XX | XX | XX |
| **Locus 22** | XY | YY | XX | YY | YY | YY | XX | YY | YY | XX | YY | YY | YY | YY | YY | YY | YY | YY | XX | YY | YY | YY |
| **Locus 23** | YY | YY | YY | YY | YY | YY | YY | YY | YY | YY | YY | YY | YY | YY | YY | YY | YY | YY | YY | YY | YY | YY |
| **Locus 24** | XY | XX | XX | XX | XX | YY | YY | XX | XX | YY | YY | XX | XX | XX | XX | XX | XX | XX | YY | XX | XX | XX |

### Example 7: Production of Di-Haploid Cucumis sativus and Cucurbita pepo Plants through Androgenesis

### a) This is a prophetic example.

### b) Flower bud selection, preparation and anther extraction

Cucumber and squash plants were cultivated either in a controlled environment or in the field, as described in Example 1. Flower buds approximately 5 mm in length - representing the optimal development stage - were collected and placed in resealable bags containing a damped paper towel. Samples were transferred in a cooler with freezer packs. Upon arrival in the lab, the flower buds were first disinfected with 70% ethanol for two min, followed by three rinses with sterile water. Subsequently, they were surface-sterilized in a 1.0-1.4% sodium hypochlorite (prepared from 20% Clorox Ultra) with agitation at a speed of 150 rpm for 10 minutes. The buds were then rinsed three additional times with sterile water. All the subsequent steps were performed under aseptic conditions.

### c) Anther culture for embryogenesis

The anther filaments were completely removed, and five anthers were placed on anther induction medium comprising Murashige & Skoog Basal Medium with Vitamins (PhytoTech Labs, Cat. Number: M519), 800 mg/L Glutamine (Millipore Sigma Cat# 56-85-9), 100 mg/L putrescine (Millipore Sigma Cat# 110-60-1), 1 mg/L 2,4-D, and 100 g/L sucrose. Depending on the genotype, the anthers were incubated in the dark at 35°C for 2-7 days. Subsequently, they were transferred to a 25°C growth room under a 16-hour light/ 8-hour dark photoperiod. The cultured anthers were transferred to freshly prepared anther induction medium every 16-20 days until callus has emerged.

The callus was carefully excised from anther and placed on germination medium (Murashige & Skoog Basal Medium with Vitamins (PhytoTech labs, M519); 100 g/L sucrose, 800mg/L Glutamine, 1 mg/L 2,4-D, 0.5 mg/L BAP, and 0.7% agar) to induce organogenesis.

The green organogenic nodules will become visible within 3-6 weeks after transferring the callus to the germination medium. Approximately 20 days later, these nodules will develop into primordium-like structures from which shoots subsequently emerges.

### d) Plantlet development

The regenerated shoots are transferred to rooting medium (MS salts with 0.2 mg/L IAA, 30 g/L sucrose, and 0.7% agar). Once a well-developed root system is established, the plants are transplanted into soil using the same procedure described in Example 1.

### d). Ploidy test and genotypic analysis of the regenerated plants

Similar methods are used to determine the ploidy level of the regenerated cucumber and squash plants as described in Example 1. Briefly, the ploidy level of plantlets at the 5-10 leaf stage are assessed using flow cytometry to differentiate between diploid (2n) and haploid (n) plants, following the method described by Eeckhaut et al. (2005). Leaf samples are collected from fully expanded leaves and processed for flow cytometry analysis. The ploidy determination is conducted according to the manufacturer's protocol (Cyflow Space, Sysmex, Germany).

Genotyping of cucumber and squash double haploid plants is performed similarly as described in Example 1. Leaf samples are taken from each plant, and all the plants are numerically labelled for future pairing by genomic selection. DNA is extracted using the Wizard^{®} Magnetic 96 DNA Plant System by Promega (Madison, WI 53711). Genotyping is carried out using Fluidigm^{®} SNP Type^{™} assay, a high-throughput, low-cost SNP genotyping method based on allele-specific PCR SNP (single-nucleotide polymorphism) detection chemistry. This approach enables rapid assay design and efficient polymorphism screening. Sequencing data is used to identify polymorphism within the genome of cucumber hybrids. Based on sequencing data, a panel of SNP markers is selected for genotyping cucumber or squash double haploid plants.

### Example 8: Production of Di-Haploid Daucus carota Plants Through Androgenesis

### a) This is a prophetic example.

### b) Flower bud selection, preparation and anther extraction

Carrot plants are grown in the controlled environment or in the field as described in Example 1. Umbels (inflorescences consisting of flower clusters with short flower stalks) are harvested at an optimal stage of development to maximize the number of microspores at the late uninucleate stage. Carrot flower buds from the two outer rows of umbels are sterilized in 96% ethanol for 60 seconds, followed by soaking in 1% sodium hypochlorite for 20 minutes. The flower buds are then washed three times in sterile distilled water. Anthers are carefully dissected from the flower buds under sterile conditions. Approximately 50 anthers are placed in the induction basal medium comprising B5 basal medium (Cat. No. G398, PhytoTech Labs, Lenexa, KS 66215) with various combinations of cytokinins and auxins, and 100 g/L sucrose. It is critical to handle the anthers gently to prevent damage and avoid unwanted somatic callus formation.

### c) Plantlet regeneration

The anther culture is maintained in darkness at 27-37°C. Embryos emerge approximately six weeks after initiation. Once they appear, the embryos are transferred to continuous light conditions while maintaining the same temperature. When the embryos begin to turn green, they are transferred to B5 germination medium comprising B5 basal medium (Cat. No. G398, PhytoTech Labs, Lenexa, KS 66215), 500 mg/L L-glutamine, 100 mg/L L-serine, 20 g/L sucrose, and 0.5% agar, and kept under fluorescent light at 30 µmol m⁻² S⁻¹, 20-25°C, with a 16-hour light/ 8-hour dark photoperiod.

Once the embryos germinate into plantlets, they are transferred to jars containing the same B5 rooting medium. The plantlets remain in this medium until a fully developed root system is established.

### d) Ploidy test and genotypic analysis of the regenerated plants

Similar methods are used to determine the ploidy level of the regenerated carrot plants as described in Example 1. Briefly, the ploidy level of plantlets at the 5-10 leaf stage are assessed using flow cytometry to differentiate between diploid (2n) and haploid (n) plants, following the method described by Eeckhaut et al. (2005). Leaf samples are collected from fully expanded leaves and processed for flow cytometry analysis. The ploidy determination is conducted according to the manufacturer's protocol (Cyflow Space, Sysmex, Germany).

Genotyping of carrot double haploid plants is performed similarly as described in Example 1. Leaf samples are taken from each plant, and all the plants are numerically labelled for future pairing by genomic selection. DNA is extracted using the Wizard^{®} Magnetic 96 DNA Plant System by Promega (Madison, WI 53711). Genotyping is carried out using Fluidigm^{®} SNP Type^{™} assay, a high-throughput, low-cost SNP genotyping method based on allele-specific PCR SNP (single-nucleotide polymorphism) detection chemistry. This approach enables rapid assay design and efficient polymorphism screening. Sequencing data is used to identify polymorphism within the genome of carrot hybrids. Based on sequencing data, a panel of SNP markers is selected for genotyping carrot double haploid plants.

### Example 9: Efficient Production of DH/Di-Haploid Allium cepa L. Plants through Gynogenesis

### a) This is a prophetic example.

### b) Flower bud selection, preparation and ovary extraction

The bulbs of donor plants are planted in the field under natural conditions, or in plastic pots in a controlled environment at a temperature of 18-25°C. Umbels are harvested when 20-40% of the flowers are at three to four days before anthesis. Flowers measuring 3-5 mm in length from each umbel are selected and sterilized by soaking in 70% ethanol for two minutes, followed by 1% sodium hypochlorite for 20 minutes. The flower buds are then rinsed at least three times with sterile distilled water.

The flower buds are subjected to heat stress treatment at 35°C for 4-10 days, 1) to eliminate maternal tissue to prevent somatic cell regeneration; and 2) to promote the transition from gametophytic to sporophytic development and double haploid cell growth. Following the stress treatment, flower buds are cultured on onion induction medium (Table 1-1, Dunstan and Short, 1977) with different combinations of cytokinins and auxins such as 2,4-D, IAA, kinetin, BAP and Zeatin until embryo production.

### c) Plant regeneration, acclimation and ploidy analysis

Onion plantlets emerge approximately 8 weeks after culture. They are then transferred to a rooting medium (half-strength MS salts, 30 g/L sucrose, and 5 g/L agar). Once the plants reach the 3-4 leaf stage with an established root system, their ploidy levels are determined using flow cytometry as described in Example 1.

It is understood that the foregoing detailed description and accompanying examples are merely illustrative and are not to be taken as limitations upon the scope of the disclosure, which is defined solely by the appended claims and their equivalents.

Various changes and modifications to the disclosed embodiments will be apparent to those skilled in the art. Such changes and modifications, including without limitation those relating to the chemical structures, substituents, derivatives, intermediates, syntheses, compositions, formulations, or methods of use of the disclosure, may be made without departing from the spirit and scope thereof.

### REFERENCE

Calabuig-Serna, A., Porcel, R., Corral-Martinez, P., Segui-Simarro, J.M. (2021) Anther and Isolated Microspore Culture in Eggplant (Solanum melongena L.). Methods in Molecular Biology 2288:235-250
Chaikam, V., Molenaar, W., Melchinger, A.E., Boddupalli, P. M. (2019) Doubled Haploid Technology for Line Development in Maize: Technical Advances and Prospects. Theoretical and Applied Genetics 132:3227-3243*.*
Charlesworth, D., Willis, J. H. (2009) The genetics of inbreeding depression. Nat Rev Genet. 10 (11):783-796.
Dunstan, D., Short, K.C. (1977) Improved growth of tissue cultures of the onion, Allium cepa. Physiologia Plantarum 41:70-72.
Eeckhaut, T., Leus, L., Van Huylenbroeck, J. (2005) Exploitation of Flow Cytometry for Plant Breeding. Acta Physiologiae Plantarum 27:743-750*.*
Flint-Garcia, S. A., Buckler, E. S., Tiffin, P., Ersoz, E., Springer, N. M. (2009) Heterosis is prevalent for multiple traits in diverse maize germplasm. PLoS One 4(10), e7433.
Esin, A. (2021) Shed-Microspore Culture in Ornamental Peppers for Doubled Haploid Plant Production. Double Haploid Technology 2: 251-265.
Grozeva, S., Pasev, G., Radeva-Ivanova, V., Todorova, V., Ivanova, V., Nankar, A. (2021) Double Haploid Development and Assessment of Androgenic Competence of Balkan Pepper Core Collection in Bulgaria. Plants 10:2414-2429.
Hamazaki, K., Iwata, H. (2024) AI-Assisted Selection of Mating Pairs through Simulation-Based Optimized Progeny Allocation Strategies in Plant Breeding. Frontiers in Plant Science 15:1-13*.*
Kele 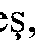 , D., Pmar, H., Ata, A., Ta 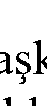 km, H.,Yildiz, S., Büyükalaca, S. (2015) Effect of Pepper Types on Obtaining Spontaneous Doubled Haploid Plants via Anther Culture. *HortScience* 50 (11):1671-1676.
Knott, L.S., Polsoni, L., Beversdorf, W.D. (1988) Cytological Aspects of Isolated Microspore Culture of Brassica napus. Can. J. of Botany 66(8):1658-1664.
Murashige, T, Skoog, F. (1962) A Revised Medium for Rapid Growth and Bio Assays with Tobacco Tissue Cultures. Physiologia Plantarum. 15 (3):473-497.
Ren, J., Wu, P., Trampe, B., Tian, X., Lubberstedt, T., Chen, S. (2017) (Novel techniques in Doubled Haploid Line Development. Plant Biotechnol. J. 15(11):1361-1370.
Segu-Simarro, J. M., Jacquier, N.M.A., Widiez, T. (2021) Overview of In Vitro and In Vivo Doubled Haploid Technologies. Methods Mol. Biol. 2287:3022.
Tomkowiak, A., Bocianowski, J., Kwiatek, M., Kowalczewski, P. L. (2020) Dependence of the Heterosis Effect on Genetic Distance, Determined using Various Molecular Markers. Open Life Sci. 15:1-11.
Tuvesson, S., Ljungberg, A., Johansson, N., Karlsson, K.E., Suijs, L.W., Josset, J.P. (2000) Large-Scale Production of Wheat and Triticale Doubled Haploids Through the Use of A Single Anther Culture Method. Plant Breeding 119(6):455-4594.
Weller, C. A., Tilk, S., Rajpurohit, S., Bergland, A. O. (2021) Accurate, ultra-low coverage genome reconstruction and association studies in Hybrid Swarm mapping populations. G3 Genes|Genomes|Genetics, 11(7)*.*
Xu, C., Wu, K., Zhang, J. G., Shen, H., Deng, H. W. (2016) Low-, high-coverage and two-stage DNA sequencing in the design of the genetic association study. Genetic Epidemiology, 41(3):187-197*.*
Yang, J., Mezmouk, S., Baumgarten, A., Buckler, E. S., Guill, K. E., McMullen, M. D., Mumm, R. H., Ross-Ibarra, J. (2017) Incomplete dominance of deleterious alleles contributes substantially to trait variation and heterosis in maize. PLoS Genet 13(9), e1007019.
Yao, H., Dogra Gray, A., Auger, D. L., Birchler, J. A. (2013) Genomic dosage effects on heterosis in triploid maize. Proc Natl Acad Sci U S A, 110(7):2665-2669.

## Claims

1. A method for producing a di-haploid *Solanaceae* plant from microspores, the method comprising:
a) selecting one or more *Solanaceae* plant flower buds having a length of about 2 to about 6 mm;
b) excising one or more anthers containing microspores from the flower buds;
c) subjecting the anthers containing microspores to one or more stress conditions selected from the group consisting of heat treatment, osmotic treatment, or a combination of heat and osmotic treatments;
d) culturing or growing anthers containing microspores on an induction medium to allow: (i) the microspores to develop into plants; and (ii) for spontaneous chromosome doubling, wherein the chromosome doubling results in di-haploid plants;
e) selecting the di-haploid *Solanaceae* plants; and
f) growing the selected di-haploid *Solanaceae* plants to maturity and collecting seed.

2. The method according to claim 1, wherein the induction medium comprises at least: a) one or more ammonium salts; b) calcium chloride; c) boric acid; d) cupric sulfate; e) magnesium sulfate; f) manganese sulfate; g) molybdic acid (sodium salt); h) potassium nitrate; i) potassium phosphate or sodium phosphate; j) zinc sulfate; k) myo-inositol; 1) nicotinic acid; m) pyridoxine HCl; n) thiamine HCl; and o) one or more plant growth regulators selected from the group consisting of: (i) 0.1-1 mg/L IAA and 0.05-2 mg/L zeatin; (ii) 0.1 mg/L 2,4-D, 0.88 mg/L IAA, and 0.55 mg/L zeatin; (iii) 0.02-5 µM 2,4-D; (iv) 5 mg/L kinetin and 5 mg/L 2,4-D; and (v) 0.01-0.5 mg/L thidiazuron (TDZ) and 1-10 mg/L zeatin..

3. The method according to claim 2, wherein the ammonium salt is ammonium nitrate or ammonium sulfate.

4. The method according to any one of claims 1-3, wherein:
i) the microspores are at a late uninucleate to early binucleate development stage;
ii) the heat treatment is carried out at about 26-40°C for 1-40 days in the dark;
iii) the osmotic treatment comprises supplying about 100 to about 120 g/L of sucrose to the induction medium
iv) selecting of di-haploid *Solanaceae* plants comprises using flow cytometry or genotyping;
v) the *Solanaceae* plant is selected from the group consisting of tomato, pepper and eggplant; and/or
vi) the medium is a liquid medium, a semi-solid medium, or a combination comprising a semi-solid layer and a liquid layer.

5. The method according to any one of claims 1-4, wherein the heat treatment is carried out at about 27-37°C for 3-10 days in the dark.

6. A di-haploid *Solanaceae* plant, plant part, plant seed, or plant embryo produced by the method according to any one of claims 1-5, wherein the di-haploid plant is homozygous at substantially all genetic loci and is free of chimeric tissues.

7. A method for producing a di-haploid *Cucurbitaceae* plant from ovaries or ovules, the method comprising:
a) selecting and collecting unfertilized ovaries from a *Cucurbitaceae* plant about six hours before anthesis;
b) slicing the ovaries into about 1-5 mm cross sections;
c) subjecting the slices to one or more stress conditions selected from the group consisting of heat treatment, osmotic treatment, or a combination of heat and osmotic treatments;
d) culturing or growing the ovary slices on an induction medium to allow: (i) development into plants; and (ii) for spontaneous chromosome doubling, wherein the chromosome doubling results in di-haploid *Cucurbitaceae* plants;
e) selecting the di-haploid *Cucurbitaceae* plants; and
f) growing the selected di-haploid *Cucurbitaceae* plants to maturity and collecting seed.

8. The method according to claim 7, wherein the induction medium comprises at least a) one or more ammonium salt; b) calcium chloride; c) boric acid; d) cupric sulfate; e) magnesium sulfate; f) manganese sulfate, g) molybdic acid (sodium salt); h) potassium nitrate; i) potassium phosphate or sodium phosphate; j) zinc sulfate; k) myo-inositol; 1) nicotinic acid; m) pyridoxine HCl; n) thiamine HCl; and/or o) 0.01-0.5 mg/L thidiazuron (TDZ).

9. The method according to claim 7 or 8, wherein:
i) the ammonium salt is ammonium nitrate or ammonium sulfate;
ii) the heat treatment is carried out at about 26-40°C for 1-60 days in the dark;
iii) the selecting comprises using flow cytometry, genotyping, or low coverage sequencing; and/or
iv) the *Cucurbitaceae* plant is selected from the group consisting of zucchini, cucumber, pumpkin, and melon.

10. The method according to any one of claims 7-9, wherein the heat treatment is carried out at 27-37°C for 1-42 days in the dark.

11. A di-haploid *Cucurbitaceae* plant, plant part, plant seed, or plant embryo produced by the method according to any one of claims 7-9, wherein the di-haploid plant is homozygous at substantially all genetic loci and is free of chimeric tissues.

12. A method for accelerated selection of new commercial hybrid vegetable varieties, the method comprising:
a) selecting elite F1 hybrid plants with broad genetic backgrounds and/or complementary agronomic traits;
b) crossing the select5ed F1 hybrid plants to produce F2 populations;
c) selecting top-performing F2 plants and performing whole genome sequencing to generate marker panel;
d) producing di-haploid plants from the selected F2 plants using the methods according to any one of claims 1-5 or claims 7-9;
e) subjecting the produced di-haploid plants to genotyping using heterozygous markers generated from the marker panel;
f) selecting di-haploid lines with beneficial allele combinations using AI-assisted genomic selection;
g) crossing the selected di-haploid lines to produce hybrid candidates; and
h) validating heterosis performance in the hybrid candidates through field trials.

13. The method according to claim 12 wherein the selecting elite F1 hybrid plants in step a) comprises whole genome sequencing.

14. The method according to claim 12 or claim 13, wherein the agronomic traits are selected from the group consisting of yield, fruit quality, disease resistance, and stress tolerance, or any combination thereof.
